# EUROPEAN PATENT SPECIFICATION

(11) **EP 2 033 655 B1**
(45) Date of publication and mention of the grant of the patent: **10.11.2010**
(21) Application number: 07301337.7
(22) Date of filing: 04.09.2007
(51) Int. Cl.: A61K 38/10, A61K 38/02, A61K 39/395, A61K 39/09, C07K 7/00, C12Q 1/00, A61P 31/04

(54) **Use of compounds that inhibit the EF-Tu-nucleolin interaction for preventing or treating tularemia**
Verwendung von Verbindungen, die die EF-Tu-Nucleolin Wechselwirkung vehindern, zur Verhinderung oder Behandlung von Tularämie
Utilisation de composés qui inhibent l'interaction EF-Tu-Nucleoline pour prévenir ou traiter la tularémie

(43) Date of publication of application: 11.03.2009
(73) Proprietor: INSERM (Institut National de la Santé et de la Recherche Médicale), 75654 Paris Cedex 13 (FR)
(72) Inventor: Barel, Monique - INSERM U570, 156 rue de Vaugirard, 75730 Paris Cedex 15 (FR); Charbit, Alain - INSERM U570, 156 rue de Vaugirard, 75730 Paris Cedex 15 (FR); Hovanessian, Ara - UPR 2228 CNRS, 45 rue des Saints-Pères, 75270 Paris Cedex 06 (FR); Briand, Jean-Paul - UPR 9021 CNRS, 15 rue René Descartes, 67084 Strasbourg Cedex (FR)
(74) Representative: Holtz, Béatrice

(56) References cited:
- WO-A-98/14587
- WO-A-2004/003009
- WO-A-2005/035579
- PETROSINO J.F. ET AL.: "Chromosome rearrangement and diversification of Francisella tularensis revealed by the type B (OSU18) genome sequence." J. BACTERIOL., vol. 188, no. 19, October 2006 (2006-10), pages 6977-6985, XP002469054
- DATABASE WPI Week 2006 Derwent Publications Ltd., London, GB; AN 2006-812332 XP002469055 & SU 1 839 960 A (GAMALEI APIDEMIOLOG. MICROBIOLOG. RES. INST.) 20 June 2006 (2006-06-20)
- NISOLE SEBASTIEN ET AL: "The anti-HIV pentameric pseudopeptide HB-19 binds the C-terminal end of nucleolin and prevents anchorage of virus particles in the plasma membrane of target cells" JOURNAL OF BIOLOGICAL CHEMISTRY, AMERICAN SOCIETY OF BIOLOCHEMICAL BIOLOGISTS, BIRMINGHAM,, US, vol. 277, no. 23, 7 June 2002 (2002-06-07), pages 20877-20886, XP002414667 ISSN: 0021-9258
- JANOVSKA ET AL: "Identification of immunoreactive antigens in membrane proteins enriched fraction from Francisella tularensis LVS" IMMUNOLOGY LETTERS, AMSTERDAM, NL, vol. 108, no. 2, 9 February 2007 (2007-02-09), pages 151-159, XP005876101 ISSN: 0165-2478

## Description

### FIELD OF THE INVENTION :

The present invention relates to methods for preventing or treating Tularemia. The methods involve inhibiting the interaction between Elongation Factor Tu (EF-Tu) of *Francisella tularensis,* and nucleolin.

### BACKGROUND OF THE INVENTION :

*Francisella tularensis* (*F. tularensis*) is a small non-motile Gram-negative bacterium that causes the zoonotic disease tularemia in a large number of animals, such as rabbits, hares, and small rodents. The disease is endemic in North America, and parts of Europe and Asia. The disease is named after Tulare County, California, and is also known as "rabbit fever". In the United States, although records show that tularemia was never particularly common, incidence rates have further dropped to below 0.1 per 100,000, meaning the disease is extremely rare in the US today.

*F. tularensis* is also one of the most infectious human bacterial pathogens. Humans acquire infection by direct contact with sick animals, inhalation, ingestion of contaminated water or food, or by bites from ticks, mosquitoes or flies. *F. tularensis* has significant potential as an agent of bioterrorism due to its infectivity and capacity to infect and its ability to cause illness and death (Dennis, D *et al*., 2001). It is one of the most infectious bacteria known for causing disease in humans (Dennis, D *et al.,* 2001; Sjostedt, A. 2003)..

Five subspecies (ssp) of *F. tularensis* have been reported: *F. tularensis* ssp tularensis (also referred as type A), *F. tularensis* ssp holarctica (type B), *F. tularensis* ssp mediasiatica, *F. tularensis* ssp novicida and *F. tularensis ssp philomiragia.* These five subspecies differ in terms of their pathogenicity and geographic origin, but are phylogenetically very closely related. *F. tularensis* is a highly virulent facultative intracellular bacterium, replicating intracellularly and disseminating within host mononuclear phagocytes. After entry of the organism into macrophages, *F. tularensis* initially resides in the phagosome, whose maturation is then arrested. After longer time of infection, the phagosome membrane is disrupted and the bacterium replicates freely in the cytoplasm of the macrophages (Golovliov, I. *et al*., 2003; Clemens, D. L. *et al*., 2004). While aspects of intracellular life after entry have been reported, the exact mechanisms that mediate uptake of this highly infectious bacterium have not been reported.

Due to its high infectivity, the bacterium is of major concern to public health officials as a possible biological weapon. In the event of an intentional exposure such as in a bioterrorist attack, inhalation of aerosols can result in devastating consequences with much casualty. Therefore, it is very important for both medical professionals and public health officials to have means for treating infections caused by such a bacterium.

A few strategies have already been proposed for curing or preventing diseases due to *F. tularensis.* Although a live vaccine is available, it has not been licensed for commercial use for safety reasons. Alternatively, patent application WO2004/003009 describes induction of a protective immune response by administration of proteins and nucleic acids from *F. tularensis.* Patent application SU1839960 suggests that an attenuated strain *F. tularensis* of holarctica subspecies could be obtained to producing a vaccine.

It remains a need for an efficient vaccine and treatment in the context of a bioterrorist attack.

### SUMMARY OF THE INVENTION ;

The inventors have now identified bacterial elongation factor Tu (EF-Tu) as the key protein of *F. tularensis* that participates in eukaryotic cell recognition, binding and internalization of the bacterium, through interaction with its cognate cellular receptor protein, nucleolin.

The inventors have further shown that an inhibitor of the interaction between EF-Tu and nucleolin is useful for preventing or treating tularemia.

The invention thus relates to the use of an inhibitor of the interaction between the bacterial elongation factor Tu (EF-Tu) of *Fransiciella tularensis* and nucleolin for the manufacture of a medicament for preventing or treating tularemia.

The inhibitor may be a ligand of nucleolin, such as pseudopeptide HB-19, or an analogue thereof, or a ligand of the bacterial elongation factor Tu (EF-Tu) of *Francisella tularensis*, such as an antibody directed against EF-Tu.

The invention further relates to the use of bacterial elongation factor Tu (EF-Tu) of *F. tularensis* for the manufacture of an immunogenic composition for preventing or treating tularemia. For that purpose the invention provides an immunogenic composition comprising bacterial elongation factor Tu (EF-Tu) of *F. tularensis.*

Another subject of the invention is a pharmaceutical composition comprising an antibody against bacterial elongation factor Tu (EF-Tu) of *F. tularensis.*

The invention further provides an in vitro method for screening compounds useful for preventing or treating tularemia, comprising contacting a compound with bacterial elongation factor Tu (EF-Tu) of *F. tularensis* and nucleolin or the RGG domain of nucleolin, and determining the ability of the compound to inhibit the interaction between EF-Tu and nucleolin or the RGG domain of nucleolin, wherein inhibition of the interaction is indicative of a compound useful for preventing or treating tularemia.

### DETAILED DESCRIPTION OF THE INVENTION:

The inventors first showed that EF-Tu is surface-exposed in *F. tularensis* live vaccine strain (LVS). They evaluated its role in bacterial adhesion and entry, using non-adherent cells of a human monocytic cell line.

They also demonstrated that nucleolin, which is present on the monocytic cell surface, disappears from cell surface following infection by LVS. Then, they showed that nucleolin, expressed on cell surface, co-localized with EF-Tu expressed at the surface of LVS after engulfment of the bacteria. In contrast, when nucleolin is detected in the nucleus, co-localization is no longer observed. Furthermore, using a pull-down assay, they showed that nucleolin directly interacted with the recombinant EF-Tu protein.

More particularly, EF-Tu was shown to bind the last C-terminal tail of nucleolin that contains the motif RGG (Arginine-Glycine-Glycine), which is repeated nine times. This C-terminal tail of nucleolin composed of 63 amino acid residues is referred to as the RGG domain of nucleolin. The RGG domain is the site in nucleolin where an inhibitor of the interaction between EF-Tu and nucleolin (pseudopeptide HB-19) binds. In the case of EF-Tu, the inventors showed that purified EF-Tu binds in a dose dependent and specific manner the synthetic 63 amino acid peptide corresponding to this RGG domain, thus indicating that the RGG domain is the site in nucleolin where *F. tularensis* EF-Tu binds.

HB-19 binds the RGG domain of nucleolin in an irreversible manner, hence preventing the binding of EF-Tu to the RGG domain, and thus to nucleolin.

Finally, the inventors showed that the use of nucleolin-specific pseudopeptide HB-19 provides an attractive therapeutic approach for modulating *F. tularensis* infection. Indexed, pre-incubation of monocytes with HB-19 pseudopeptide inhibited both the binding and the infection of monocytes by LVS.

### Definitions:

An "inhibitor" of the interaction is a molecule, which can block the interaction between two molecules or more by competition or by fixing in one of the molecule.

By "ligand" is meant a natural or synthetic compound which binds a receptor molecule to form a receptor-ligand complex. In the present invention, the ligand is preferably an antagonist of the receptor.

The term "small organic molecule" refers to a molecule of a size comparable to those organic molecules generally used in pharmaceuticals. The term excludes biological macromolecules (e. g. , proteins, nucleic acids, etc.). Preferred small organic molecules range in size up to about 5000 Da, more preferably up to 2000 Da, and most preferably up to about 1000 Da.

By "purified" or "isolated" is meant, when referring to a polypeptide or a nucleotide sequence, that the indicated molecule is present in the substantial absence of other biological macromolecules of the same type. The term "purified" as used herein preferably means at least 75% by weight, more preferably at least 85% by weight, still preferably at least 95% by weight, and most preferably at least 98% by weight, of biological macromolecules of the same type are present. An "isolated" nucleic acid molecule, which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

According to the present invention, "antibody" and "immunoglobulin" have the same meaning, and will be used equally in the present invention. In natural antibodies, the two heavy chains are linked to each other, by disulfide bonds and each heavy chain is linked to a light chain by a disulfide bond. There are two types of light chain, lambda (I) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin consisting of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) influence the overall domain structure and hence the combining site. Complementarity Determining Regions or CDRs refer to amino acid sequences, which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs.

A "monoclonal antibody" or "mAb" in its various grammatical forms refers to a population of antibody molecules that contains only one species of antibody combining site capable of immunoreacting with a particular epitope. A monoclonal antibody thus typically displays a single binding affinity for any epitope with which it immunoreacts

The term "hybridoma" denotes a cell, which is obtained by subjecting a B cell prepared by immunizing a non-human mammal with an antigen to cell fusion with a myeloma cell derived from a mouse or the like, which will produce a desired monoclonal antibody having an antigen specificity.

### Elongation factor:

The term Elongation Factor Tu (EF-Tu) denotes a protein factor, which is essential for the elongation of the polypeptide chain at the time of the translation of proteins (in both eukaryotes and prokaryotes). EF-Tu allows the binding of codon-specific aminoacyl-tRNA to the ribosome aminoacyl or-site, the first step in the process of peptide chain elongation. It is an abundant protein, which exists in two forms binding GTP and GDP respectively (Genbank Access Number: CAJ80190). An amino acid sequence of EF-Tu of *F. tularensis* is shown as SEQ ID NO:1.

Recent studies have unraveled an unexpected key role of EF-Tu in microbial pathogenesis. EF-Tu is located at the cell surface of *Lactobacillus johsonii* and mediates its attachment to human intestinal cells and mucins (Gronato *et al.,* 2004); EF-Tu is one of the six most abundant mycobacterial proteins, whose expression increases after phagocytosis by macrophages (Monahan *et al*., 2001). Furthermore EF-Tu of *Listeria monocytogenes* is a target for a serine-threonine phosphatase, which is involved in virulence of this pathogen (Archambaud *et al.,* 2005). EF-Tu is also present in purified *Anaplasma marginale* outer membranes that induces complete protection against infection (Lopez *et al*., 2005). At last, EF-Tu is used for specific serodiagnosis of contagious bovine pleuropneumonia (Alonso *et al.,* 2002). Moreover, analysis of the anti- *F. tularensis* immunoproteome identified EF-Tu as one of the major antigenic protein of LVS, in the murine model of infection (Havlasova *et al*., 2005) and as an immunoreactive antigen in the membranes of LVS in human infection (Janovska *et al*., 2007).

### Nucleolin:

The term "Nucleolin" denotes an ubiquitous protein which is encoded by a gene localized on chromosome 2q12-qter on human Genome with the characteristic GC-rich promoter sequences found in housekeeping genes (Srivastava M., *et al*., 1990).

Nucleolin is a protein that is able to translocate from cell surface to the cytoplasm, (Hovanessian *et al*., 2000). Nucleolin, which is involved in cell proliferation (Srivastava and Pollard, 1999), has been previously described as a receptor for the adhesin of *E. coli* O157:H7 (Robinson *et al.,* 2006, Sinclair and O'Brien, 2002).

Nucleolin has three main structural domains: 1) the N-terminal domain containing several long stretches of acidic residues; 2) the central globular domain containing four RNA binding domains (RBDs); and 3) the extreme C-terminal domain containing nine repeats of the tripeptide motif arginine-glycine-glycine (RGG domain) (Srivastava et al, 1999). The RGG domain has been shown to be responsible for the interaction of nucleolin with various protein-ligands and even HIV particles (Nisole et al, 2002; Said et al, 2005).

An aminoacid sequence of human nucleolin is shown as SEQ ID NO:2 (Genbank Access number NP005372).

### Inhibitors

In the context of the invention, the inhibitor is preferably a ligand, which inhibits the interaction between EF-Tu and nucleolin.

The ligand may be *e.g*. a small organic molecule, an antibody, or a polypeptide.

In one embodiment said ligand is a ligand of nucleolin, which preferably binds the RGG domain of nucleolin.

The ligand may be a multivalent synthetic compound which comprises or is constituted of a template on which at least 3 pseudopeptides are grafted, said pseudopeptides having the formula 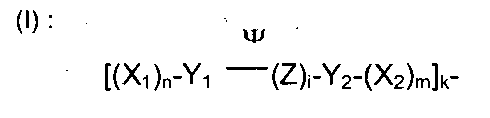 wherein
X₁ and X₂ independently represents any amino acid;
Y₁ and Y₂ independently are amino acids having a basic lateral chain;
Z is selected from the group consisting of :
   - proline, optionnally substituted in γ, β or δ by hydroxyl, amino, C₁-C₁₀ alkyl, C₁-C₁₀ alkenyl, C₁-C₁₀ alkynyl, C₅-C₁₂ aryl, C₅-C₁₄ aralkyl, C₅-C₁₂ heteroaryl group, such group being optionnally substituted by 1 to 6 substituents selected from the group consisting of halogen, NO₂, OH, C₁-C₄ alkyl, NH₂, CN, trihalomethyl, C₁-C₄ acyloxy, C₁-C₄dialkylamino, guanidino, thiol;
   - (natural or not natural) N-alkylated amino acid;
   - dialkylated amino acid;
   - dialkylated cyclic amino acid; or
   - pipecolic acid or one derivative thereof;
   n and i independently are 0 or 1 ;
   m is an integer between 0 and 3 ;
   k is an integer superior or equal to 3 ; and
   represents a modified peptide bond, significantly more resistant to at least one protease than a normal peptide bond.

Preferably, n is 0 and m is 0,1 or 2, preferably 0 or 1. In a preferred embodiment both n and m are 0.

The term « template » herein refers to any pharmaceutically acceptable molecule, i.e. without intrinsic toxicity, on which at least 3 pseudopeptides of formula (I) can be grafted. The template must thus be sufficient large to allow grafting of at least 3, preferably 3 to 8 (preferably k is an integer between 3 and 8), even more preferably 5 or 6, pseudopeptide motifs of formula (I). Preferably the template is yet small enough so that the at least 3 motifs can together interact with the RGG domain of one or several nucleolin molecules.

The template should not be immunogenic.

Said template may be advantageously selected from the group consisting of a linear peptide, a cyclic peptide, a peptoide (i.e. an oligomer of N-substituted glycine) that is linear or cyclic, a foldamer (i.e. an oligomer or polymer which has a strong tendency to adopt a compact confirmation that is well defined and predictable in solution), a linear or spherical polymer or a dendrimer (i.e. a macromolecule constituted of monomers which associate according to tree process around a plurifunctional central core), a sugar, or a nanoparticle.

The template is preferably selected from the group consisting of a linear peptide, a cyclic peptide, and a peptoide (i.e. an oligomer of N-substituted glycine) that is linear or cyclic.

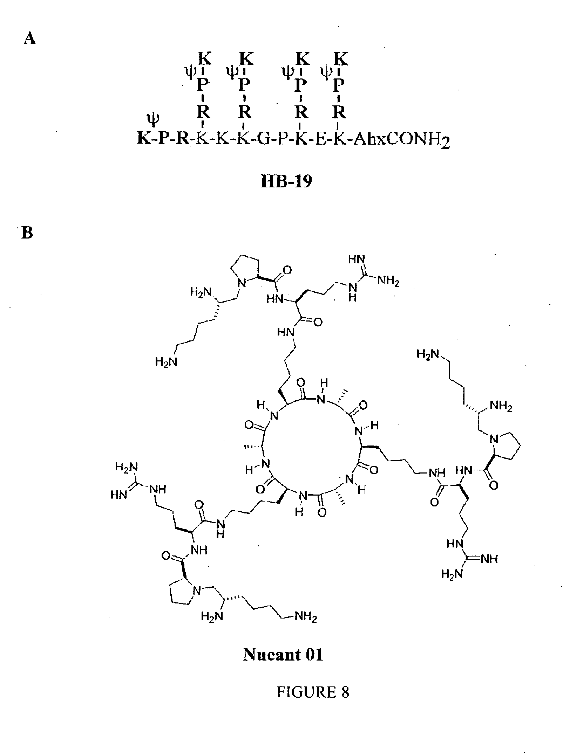

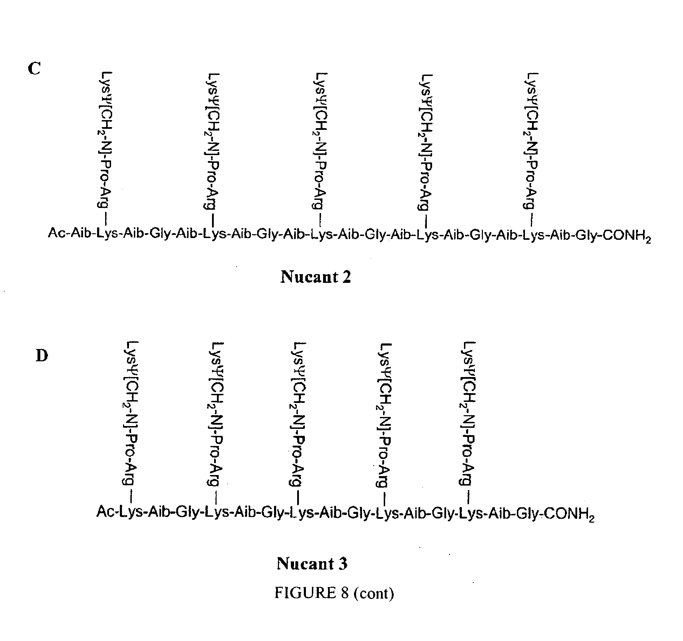

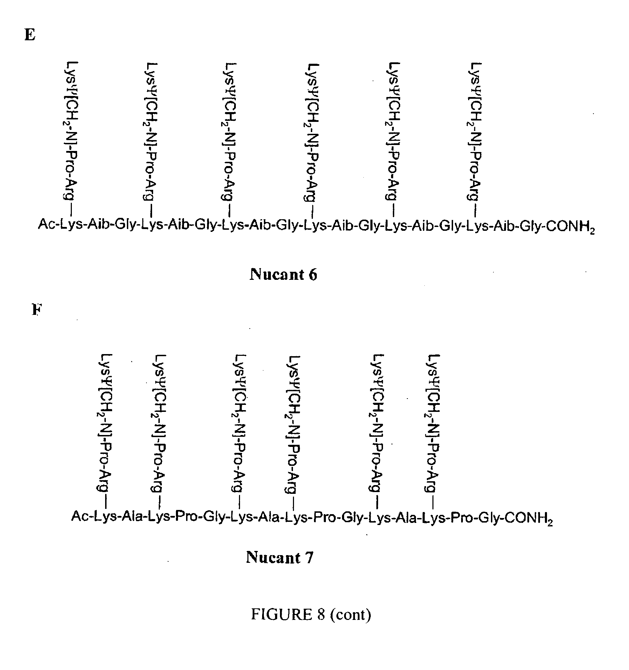

For example, a linear peptide template can have a sequence selected from the group consisting of KKKGPKEKGC (SEQ ID NO :3), KKKKGC (SEQ ID NO :4), KKKKGPKKKKGA (SEQ ID NO :5) or KKKGPKEKAhxCONH₂ (SEQ ID NO :6), wherein Ahx represents hexanoïc amino acid and CONH₂ represents an amide function instead of the acid function, AhxCONH₂ represents (2S)-2-aminohexanamide, or a linear sequence which is constituted of 2 to 4 motifs (KAKPG, SEQ ID NO :7), e.g. sequence AcKAKPGKAKPGKAKPGCONH₂ (SEQ ID NO :8), wherein Ac is acetyl CH₃-CO-, and CONH₂ meaning that the acidic function COOH from glycine is replaced by an amide function CONH₂). Advantageously the linear peptide template may be KKKGPKEKAhxCONH_{2 (}SEQ ID NO : 9) (see for example pseudopeptide HB19 on Figure 8), or peptide AcKAKPGKAKPGKAKPGCONH₂ (SEQ ID NO :10, wherein Ac is acetyl CH₃-CO-, and CONH₂ meaning that the acidic function COOH from glycine is replaced by an amide function CONH_{2,} see for example Nucant 7 on Figure 8).

Among linear peptides, some are known to adopt a helicoidal structure. For instance such peptides may comprise an integer number (at least 3, preferably from 3 to 8) of repeated motifs Aib-Lys-Aib-Gly or Lys-Aib-Gly respectively, wherein Aib represents 2-amino-isobutyric acid. Since each motif comprises one Lys residue only, as many repeats as the motifs to graft are needed.

For instance; in order to prepare a pentavalent compound with 5 pseudopeptides of formula (I), one can use a linear peptide of formula Aib-Lys-Aib-Gly-Aib-Lys-Aib-Gly-Aib-Lys-Aib-Gly-Aib-Lys-Aib-Gly-Aib-Lys-Aib-Gly (SEQ ID NO :11) ou Lys-Aib-Gly-Lys-Aib-Gly-Lys-Aib-Gly-Lys-Aib-Gly-Lys-Aib-Gly (SEQ ID NO :12).

Advantageously, a linear peptide having the formula Ac-Aib-Lys-Aib-Gly-Aib-Lys-Aib-Gly-Aib-Lys-Aib-Gly-Aib-Lys-Aib-Gly-Aib-Lys-Aib-Gly-CONH₂ (SEQ ID NO :13, wherein Ac is acetyl CH₃-CO-, and CONH₂ meaning that the acidic function COOH from glycine is replaced by an amide function CONH₂, see for example Nucant 2 on Figure 8) ou Ac-Lys-Aib-Gly-Lys-Aib-Gly-Lys-Aib-Gly-Lys-Aib-Gly-Lys-Aib-Gly-CONH₂
(SEQ ID NO :14, wherein Ac is acetyl CH₃-CO-, and CONH₂ meaning that the acidic function COOH from glycine is replaced by an amide function CONH_{2,} see for example Nucant 3 on Figure 8).

Alternatively, in order to obtain an hexavalent compound with 6 pseudopeptidic motifs of formula (I), one can use a linear sequence Ac-Aib-Lys-Aib-Gly-Aib-Lys-Aib-Gly-Aib-Lys-Aib-Gly-Aib-Lys-Aib-Gly-Aib-Lys-Aib-Gly-Aib-Lys-Aib-Gly-CONH₂ (SEQ ID NO :15, wherein Ac is acetyl CH₃-CO-, and CONH₂ meaning that the acidic function COOH from glycine is replaced by an amide function CONH₂) or Ac-Lys-Aib-Gly-Lys-Aib-Gly-Lys-Aib-Gly-Lys-Aib-Gly-Lys-Aib-Gly-Lys-Aib-Gly-CONH₂ (SEQ ID NO :16, wherein Ac is acetyl CH₃-CO-, and CONH₂ meaning that the acidic function COOH from glycine is replaced by an amide function CONH₂, see for example Nucant 6 on Figure 8).

A cyclic peptide or peptoide can be advantageously used as a template.

This allows to restrain the flexibility of the backbone.

A cyclic peptide or peptoide can be selected from the group consisting of cyclic hexa-, octa-, deca- ou dodeca- peptide preferably constituted of amino acid residues of L (levogyre) and D (dextrogyre) configuration (D,L-cyclopeptide) or of a chain of N-alkyl Glycine residues (cyclic peptoïde). An example of a compound which comprises a cyclic hexapeptide constituted by an alternance of alanine (A) in D configuration and lysine (K) in L configuration L showing 3 KPR motifs with a Ψbond (Ψ = (-CH₂N-)) between K et P is shown on Figure 8 (compound Nucant 01).

The term « grafted », with regard to pseudopeptides, means that they are linked by a colavent bond, either directly, or via a spacer molecule between the pseudopeptide motifs and the template. Examples of spacers include ethylene glycol, piperazine, or an amino acid such as aminohexanoïc acid or beta-alanine.
When the template is a cyclic or linear peptide on which pseudopeptide motifs are directly grafted, the bond usually involves the α or ε amino groups of the lysine residue on the template, and an acidic function COOH of the the C-term amino acid of the pseudopeptides.

In the context of the present invention « any amino acid » refers to a natural or synthetic amino acid, optionnally modified by one or several susbstituants. Preferably, it can be an alpha amino acid: 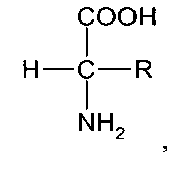 of configuration L or D, wherein R is the lateral chain of the amino acid.

The term « (C₁-Cᵢ) alkyl» means a branched or linear saturated chain -CⱼH₂ⱼ₊₁, où 1 ≤ j ≤ i, including methyl, ethyl, n-propyl, or isopropyl, n-butyl, isobutyl, sec-butyl or tert-butyl, n-pentyl, neopentyl, isopentyl, tert-pentyl, or C₆ to C₁₀ alkyl groups. The term « (C₁-C₁₀) alkenyl» means a branched or linear chain comprising from 1 to 10 Carbon atoms and at least a double bond C=C. The term «(C₁-C₁₀) alkynyl» means a branched or linear chain comprising from 1 to 10 Carbon atoms and at least a triple bond C=C. The term «(C₅-C₁₂) aryl» means a monocycle or polycycle aromatic radical having from 5 to 12 Carbon atoms. By « (C₅-C₁₄) aralkyl», is meant the combination of an alkyl and an aryl groups having a total of 5 to 14 carbon atoms. By « (C₅-C₁₂) heteroaryl » is meant an aryl group wherein at least one Carbon atom out of 5 to 12 is substituted by another atom which is N, O, or S. By «(C₁-C₄) acyloxy», is meant -O(O)C-(C₁-C₄)alkyl, -O(O)C-(C₄-C₁₂)cycloalkyl, -O(O)C-(C₄-C₁₂)aryl, - O(O)C-(C₄-C₁₂)arylalkyl or -O(O)C-(C₄-C₁₂)heteroaryl). Preferably it is selected from the group consisting of -O(O)C-(C₁-C₄)alkyl, -O(O)C-C₄ cycloalkyl, -O(O)C-C₄aryl, -O(O)C-C₄arylalkyl, or -O(O)C-C₄heteroaryl. The term « C₁-C₄ dialkylamino» means a group of formula -N(C₁-C₄alkyl)₂ wherein each alkyl group is identical or different.

The term « N-alkylated amino acid» means any amino acid wherein one H in the amino group is substituted by a C₁-C₁₀ alkyl chain or a C₅-C₁₄aralkyl chain, preferably C₅-C₁₀ aralkyl, even more preferably C₁₀ aralkyl, which chains are optionally substituted. Examples include N-methylglycine or sarcosine, N-methylisoleucine, N-methylvaline etc. By « dialkylated amino acid », is meant any amino acid wherein two hydrogen atoms (in the central carbon or in the amino group) are substituted by a C₁-C₁₀ alkyl chain or C₅-C₁₄ aralkyl, preferably C₅-C₁₀ aralkyl, even more preferably C₁₀ aralkyl, which chains are optionally substituted. Examples include 2-amino-isobutyric acid (Aib), aminocyclopropane carboxylic acid, etc.

Preferably Z is present (i is 1). Then Z is preferably a proline, which is optionally y, β or δ-substituted.

The term « amino acid having a basic lateral chain» refers to any amino acid, wherein the lateral chain R presents a pKa value that is superior to 7 (pKa(R)>7). Examples include lysine (K, pKa(R) ≈ 10,5), arginine (R, pKa(R) ≈ 12,5), and ornithine (pKa(R) ≈ 10,8). Preferably Y₁ and/or Y₂ is lysine (K) or arginine (R). More preferably Y₁ is lysine (K) and Y₂ is arginine (R).

The term « a normal peptide bond» means an amide of formula (-CONH-), which is generally present between two amino acids in a natural protein. The term « modified Ψ bond », means a bond between two amino acids whis is different from the normal amide bond (-CONH-) and is more resistant to a protease, such as N-peptidases.

In a preferred embodiment, Ψ is a reduced bond (-CH₂NH-) (or (-CH₂N-) when the bond involves a secondary amino group, e.g. when a proline is involved), or retro inverso (-NHCO-), methylene oxy (-CH₂-O-), thiomethylene (-CH₂S-), carba (-CH₂CH₂-), cetomethylene (-CO-CH₂-), hydroxyethylene (-CHOH-CH₂-), (-N-N-), E-alcene or (-CH-CH-) bond. Preferably Ψ is a (-CH₂NH-) bond.

When the nature of the amino acid bond is not explicitely mentioned, then it means that it may be a normal peptide bond or optionally a modified Ψ bond.

Examples of ligands of nucleolin useful in the invention include the compounds shown on Figure 8, i.e. HB19, Nucant 01, Nucant 2, Nucant 3, Nucant 6, or Nucant 7.

HB-19 and related analogue pseudopeptides, Nucant 3, Nucant 6 and Nucant 7, are the preferred ligands: 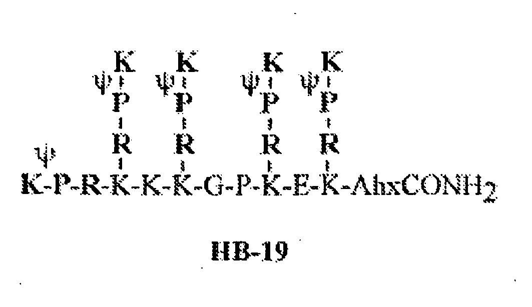 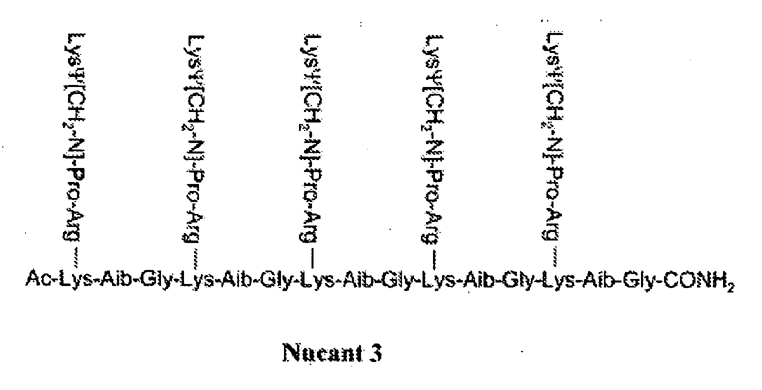 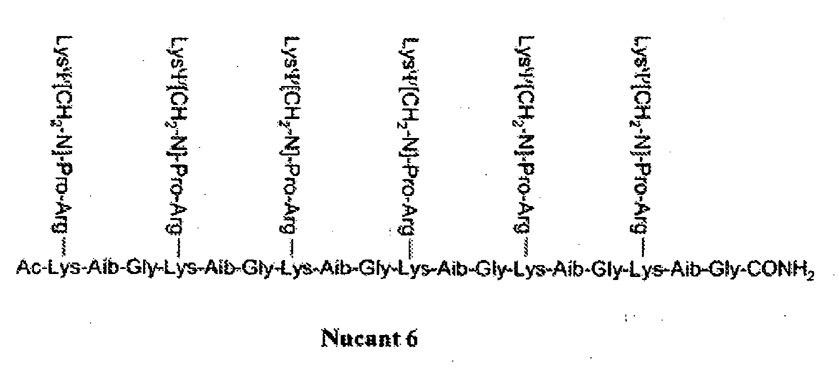 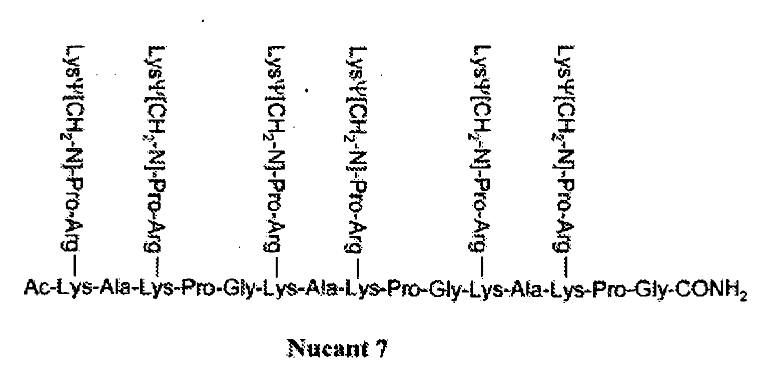 wherein ψ(CH₂-N) stands for a reduced peptide bond and AhxCONH₂ represents aminohexanamide, and Aib represents 2-amino-isobutyrique acid.

These pseudopeptides all bind specifically and irreversibly the cell-surface expressed nucleolin.

It has been shown that the HB-19 pseudopeptide and its analogues (e.g. Nucant 01, 2, 3, 6, and 7) can bind specifically and irreversibly to nucleolin and induce specific degradation of cell-surface nucleolin (Nisole *et al.,* 1999) and inhibit HIV infection (Said *et al.,* 2005).

This HB-19 pseudopeptide is also mentioned in international patent application WO98/40480, as an inhibitor of HIV attachment.

The multivalent pseudopeptide HB-19 and related analogues (PCT CNRS 2007) bind nucleolin expressed on intact cells and also nucleolin found in cell extracts. Such pseudopeptides also bind in vitro generated full-length nucleolin (containing 710 amino acid residues) but they do not bind the N-terminal part of nucleolin (amino acid residues 1-308) containing the acidic amino acid stretches. The use of various deletion constructs of the C-terminal part of nucleolin then permitted the identification of the extreme C-terminal end of nucleolin, containing repeats of the amino acid motif, RGG, as the domain that binds HB-19 and related analogues. Consequently, a synthetic peptide corresponding to the last C-terminal 63 amino acids of nucleolin (referred to as p63) binds HB-19 and related analogues. The sequence of the RGG domain p63: (amino acid residues at the C-terminal tail of nucleolin 648-710) is

These observations indicate that the binding of HB-19 and related analogues to nucleolin is not simply a matter of charge, since the binding occurs specifically at the RGG domain that does not contain acidic amino acid residues.

The pseudopeptidic molecules described above are used preferably in a soluble form.

In another embodiment, the ligand of nucleolin that inhibits the interaction between EF-Tu and nucleolin may be an antibody against the RGG domain.

In still another embodiment the inhibitor is a ligand of the EF-Tu.

Preferably, said ligand is an antibody directed against EF-Tu, preferably a monoclonal antibody, or a fragment thereof, as defined below.

Inhibitors useful in the present invention may be manufactured by the conventional process for chemical molecule synthesis.

Polypeptides of the invention may be produced by any technique known per se in the art, such as without limitation, any chemical, biological, genetic or enzymatic technique, either alone or in combination(s).

Knowing the amino acid sequence of the desired sequence, one skilled in the art can readily produce said polypeptides, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions.

Antibodies may be derived from a number of species including, but not limited to, rodent (mouse, rat, rabbit, guinea pig, hamster, and the like), porcine, bovine, equine or primate and the like. Antibodies from primate (monkey, baboon, chimpanzee, etc.) origin have the highest degree of similarity to human sequences and are therefore expected to be less immunogenic. Antibodies derived from various species can be "humanized" by modifying the amino acid sequences of the antibodies while retaining their ability to bind the desired antigen.

Procedures for raising "polyclonal antibodies" are well known in the art. For example, polyclonal antibodies can be obtained from serum of an animal immunized against the protein of interest, which may be produced by genetic engineering for example according to standard methods well-known by one skilled in the art. Typically, such antibodies can be raised, by administering the protein subcutaneously to New Zealand white rabbits, which have first been bled to obtain pre-immune serum. The antigens can be injected at a total volume of 100 µl per site at six different sites. Each injected material may contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed by (Harlow *et al*., 1988), which is hereby incorporated in the references.

Although historically a monoclonal antibody was produced by immortalization of a clonally pure immunoglobulin secreting cell line, a monoclonally pure population of antibody molecules can also be prepared by the methods of the present invention. Laboratory methods for preparing monoclonal antibodies are well known in the art (see, for example, Harlow *et al.,* 1988). Monoclonal antibodies (mAbs) may be prepared by immunizing a mammal such as mouse, rat, primate and the like, The antibody-producing cells from the immunized mammal are isolated and fused with myeloma or heteromyeloma cells to produce hybrid cells (hybridoma). The hybridoma cells producing the monoclonal antibodies are utilized as a source of the desired monoclonal antibody. This standard method of hybridoma culture is described in (Kohler and Milstein, 1975). Alternatively, the immunoglobulin genes may be isolated and used to prepare a library for screening for reactive specifically reactive antibodies. Many such techniques, including recombinant phage and other expression libraries, are known to one skilled in the art.

While mAbs can be produced by hybridoma culture the invention is not to be so limited. Also contemplated is the use of mAbs produced by cloning and transferring the nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No.; European Patent Publications No. 0239400 to Winter et al. and No. 0125023 to Cabilly *et al.*

Antibody generation techniques not involving immunisation are also contemplated such as for example using phage display technology to examine naive libraries (from non-immunised animals); see (Barbas *et al.,* 1992, and Waterhouse *et al.* (1993).

Antibodies of the invention are suitably separated from the culture medium by conventional immunoglobulin purification procedures such as, for example, protein A-Sepharose, hydroxylapatite chromatography, gel electrophoresis, dialysis, or affinity chromatography.

In a particular embodiment, the antibody of the invention may be a human chimeric antibody. Said human chimeric antibody of the present invention can be produced by obtaining nucleic sequences encoding for VL and VH domains, constructing a human chimeric antibody expression vector by inserting them into an expression vector for animal cell having genes encoding human antibody CH and human antibody CL, and expressing the expression vector by introducing it into an animal cell. As the CH domain of a human chimeric antibody, it may be any region which belongs to human immunoglobulin, but those of IgG class are suitable and any one of subclasses belonging to IgG class, such as IgG1, IgG2, IgG3 and IgG4, can also be used. Also, as the CL of a human chimeric antibody, it may be any region ,which belongs to Ig, and those of kappa class or lambda class can be used. Methods for producing chimeric antibodies involve conventional recombinant DNA and gene transfection techniques are well known in the art (See Morrison SL. *et al.* (1984) and patent documents US5,202,238; and US5,204, 244).

In another particular embodiment, said antibody may be a humanized antibody. Said humanized antibody may be produced by obtaining nucleic acid sequences encoding for CDRs domain by inserting them into an expression vector for animal cell having genes encoding a heavy chain constant region identical to that of a human antibody; and a light chain constant region identical to that of a human antibody, and expressing the expression vector by introducing it into an animal cell.

The humanized antibody expression vector may be either of a type in which a gene encoding an antibody heavy chain and a gene encoding an antibody light chain exists on separate vectors or of a type in which both genes exist on the same vector (tandem type). In respect of easiness of construction of a humanized antibody expression vector, easiness of introduction into animal cells, and balance between the expression levels of antibody H and L chains in animal cells, a tandem type of the humanized antibody expression vector is more preferable (Shitara K *et al*. 1994). Examples of the tandem type humanized antibody expression vector include pKANTEX93 (WO 97/10354), pEE18 and the like. Methods for producing humanized antibodies based on conventional recombinant DNA and gene transfection techniques are well known in the art (See, e. g., Riechmann L. *et al*. 1988; Neuberger MS. *et al*. 1985). Antibodies can be humanized using a variety of techniques known in the art including, for example, CDR-grafting (EP 239,400; PCT publication WO91/09967; U.S. Pat. Nos. 5,225,539; 5,530,101; and 5,585,089), veneering or resurfacing (EP 592,106; EP 519,596; Padlan EA (1991); Studnicka GM *et al*. (1994); Roguska MA. *et al*. (1994)), and chain shuffling (U.S. Pat. No.5, 565, 332). The general recombinant DNA technology for preparation of such antibodies is also known (see European Patent Application EP 125023 and International Patent Application WO 96/02576).

Other suitable forms of antibodies or antibody fragments may include modification of antigen binding fragments by the covalent attachment of an organic moiety, by methods well known to those of skill in the art. Such modifications can produce antibodies with increased pharmacokinetic properties (such as increase half-life in circulating blood in vivo). The organic molecule for conjugation may be selected from a number of organic polymers of molecular weight of about 1000 daltons to 120,000 daltons. Such molecules include, but are not limited to, polyethylene glycol (PEG), polypropylene glycol (PPG), carbohydrate polymer, amino acid polymer, fatty acid or fatty acid ester.

For example, antibody fragments, including, but not limited to Fab (e.g., by papain digestion), Fab' (e.g., by pepsin digestion and partial reduction) and F(ab')2 (e.g., by pepsin digestion), fab (e.g., by plasmin digestion), pFc' (e.g., by pepsin or plasmin digestion), Fd (e.g., by pepsin digestion, partial reduction and reaggregation), Fv or SCFv (e.g., by molecular biology techniques) fragments, are encompassed by the invention.

In a particular embodiment, monoclonal antibodies of the invention are monovalent, bivalent, multivalent, monospecific, bispecific, or multispecific. In another preferred embodiment, the antibody to EF-Tu is a binding fragment or a conjugate. For examples antibodies of the invention may be conjugated to a growth inhibitory agent, cytotoxic agent, or a prodrug-activating enzyme.

It may be also desirable to modify the antibody so as to render it cytotoxic with regard to the bacterial cell. This may be achieved by introducing one or more amino acid substitutions in an Fc region of the antibody. Alternatively or additionally, cysteine residue(s) may be introduced in the Fc region, thereby allowing inter-chain disulfide bond formation in this region. The homodimeric antibody thus generated may have improved internalization capability and/or increased complement-mediated cell killing and/or antibody-dependent cellular cytotoxicity (ADCC).

These modifications of the antibody do not impact its ability to interact with EF-Tu.

### Pharmaceutical compositions:

It is herein described a method of preventing or treating tularemia comprising administering in a subject in need thereof a therapeutically effective amount of an inhibitor as above described.

In the context of the invention, the term "treating" or "treatment", as used herein, means reversing, alleviating, inhibiting the progress of, or preventing the disorder or condition to which such term applies, or one or more symptoms of such disorder or condition.

According to the invention, the term "patient" or "patient in need thereof' is intended for a human or non-human mammal affected or likely to be affected with a Tularemia.

By a "therapeutically effective amount" of the inhibitors useful in the present the invention is meant a sufficient amount of the antibody to treat said disease, at a reasonable benefit/risk ratio applicable to any medical treatment. It will be understood, however, that the total daily usage will be decided, by the attending physician within the scope of sound medical judgment. The specific therapeutically effective dose level for any particular patient will depend upon a variety of factors including the severity of the disorder; the specific composition employed, the age, body weight, general health, sex and diet of the patient; the time of administration, route of administration; the duration of the treatment; drugs used in combination, etc. For example, it is well known within the skill of the art to start doses of the compound at levels lower than those required to achieve the desired therapeutic effect and to gradually increase the dosage until the desired effect is achieved.

The inhibitor useful in the present invention may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The pharmaceutical compositions of the invention can be formulated for a topical, oral, intranasal, intraocular, intravenous, intramuscular or subcutaneous administration and the like.

Preferably, the pharmaceutical compositions contain vehicles, which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, or alternatively of the desired duration of treatment.

To prepare pharmaceutical compositions, an effective amount of the inhibitor may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

In addition to the compounds formulated for parenteral administration, such as intravenous or intramuscular injection, other pharmaceutically acceptable forms include, e.g. tablets or other solids for oral administration ; time release capsules ; and any other form currently used.

Compositions of the present invention may be combined or may further comprise a further therapeutically active agent. In one embodiment said therapeutically active agent is an antibacterial agent. For example, said antibacterial agents include but are not limited to beta-lactam antibiotics, fluoroquinolones, quinolones, macrolides, and tetracyclines. Examples of fluoroquinolones include, without limitation, ciprofloxacin, clinafloxacin, enoxacin, fleroxacin, gatifloxacin, moxifloxacin, gemifloxacin, grepafloxacin, levofloxacin, norfloxacin, sparfloxacin, and trovafloxacin. Examples of quinolones include, without limitation, cinoxacin, garenoxacin, and nalidixic acid. Examples of macrolides include, without limitation, azithromycin, clarithromycin, dirithromycin, erythromycin, and lincomycin. Examples of tetracyclines include, without limitation, doxycycline, minocycline, and tetracycline.

### Screening methods:

Ligands of EF-Tu or nucleolin useful in the invention may be obtained by any screening method well known in the art.

The invention thus provides a method for screening compounds useful for preventing or treating Tularemia, comprising
- contacting a compound with bacterial elongation factor Tu (EF-Tu) of *F. tularensis* and nucleolin or the RGG domain of nucleolin, and
- determining the ability of the compound to inhibit the interaction between EF-Tu and nucleolin or the RGG domain of nucleolin,
wherein inhibition of the interaction is indicative of a compound useful for preventing or treating tularemia.

Nucleolin and EF-Tu may be in isolated form, or may be presented on cell membranes, or expressed by cells.

Suitable host cells include, without limitation, prokaryotic cells (such as bacteria) and eukaryotic cells (such as yeast cells, mammalian cells, insect cells, plant cells, etc.). However preferred host cell are yeast cells and more preferably a *Saccharomyces cerevisiae* cell or a *Schizosaccharomyces pombe* cell.

The cells that express EF-Tu may be *F. tularensis* bacterium, or a host cell transfected with EF-Tu of *F. tularensis,* such as those described above.

The RGG domain may be produced by chemical synthesis or by genetic technology via expression of its corresponding DNA sequence. Synthetic RGG domain (peptide p63) can be synthesized with a label such as biotin.

Generally the method of screening comprises generating physical values which illustrate or not the ability of said candidate compound to inhibit the interaction between said first polypeptide and said second polypeptide and comparing said values with standard physical values obtained in the same assay performed in the absence of the said candidate compound. The "physical values" that are referred to above may be of various kinds depending of the binding assay that is performed, but notably encompass light absorbance values, radioactive signals and intensity value of fluorescence signal. If; after the comparison of the physical values with the standard physical values, it is determined that the said candidate compound inhibits the binding between nucleolin and EF-Tu, then the candidate is positively selected.

The compounds that inhibit the interaction between (i) nucleolin or the RGG domain of nucleolin and (ii) EF-Tu encompass those compounds that bind either to nucleolin (or to the RGG domain of nucleolin) or to EF-Tu, provided that the binding of the said compounds of interest then prevents the interaction between nucleolin or the RGG domain of nucleolin and EF-Tu.

In a particular embodiment, the screening method may comprise the steps consisting of :
(a) contacting EF-Tu and nucleolin or the RGG domain of nucleolin with a ligand known to bind specifically to EF-TU or nucleolin or the RGG domain of nucleolin;
(b) contacting the mixture of step (a) with a candidate compound;
(c) comparing the binding of the ligand known to bind to EF-Tu or nucleolin or the RGG domain of nucleolin in the presence of said candidate compound, to the binding of ligand known to bind to EF-Tu or nucleolin or the RGG domain of nucleolin in the absence of said candidate compound, and
(d) selecting positively the candidate compound that reduces the binding of the ligand known to bind to EF-Tu or nucleolin or the RGG domain of nucleolin.

Another suitable in vitro screening method according to the invention can be carried out using labeled candidate compounds which are then incubated with a polypeptide that has a EF-Tu or nucleolin ligand binding domain. Labels include radioisotopes, immunochemicals, fluorophores, and the like. Those of skill in the art will recognize a variety of ways of separating the bound labeled candidate therapeutic agent from the free labeled candidate therapeutic agent. The affinity of the labeled candidate therapeutic agent for a EF-Tu or nucleolin or the RGG domain of nucleolin can be calculated using standard ligand binding methods.

Another type of screening method according to the invention may conducted, for example, as a direct binding assay with a labeled EF-Tu or ligand of nucleolin or of the RGG domain of nucleolin in the presence of a candidate therapeutic agent. The assays involve placing the test compound into an assay mixture that includes at least a ligand binding domain of a EF-Tu or Nucleolin polypeptide and a ligand for EF-Tu or nucleolin or the RGG domain of nucleolin. The effect on binding of ligand to EF-Tu or nucleolin or the RGG domain of nucleolin is determined. A suitable technique that can be used in said screening methods may be the Homogeneous Time Resolved Fluorescence (HTRF) assay, such as described in document WO 00/01663 or US6,740,756.

### Labelled polypeptides :

In one embodiment, nucleolin or the RGG domain of nucleolin or EF-Tu is labelled with a detectable molecule.

According to the invention, said detectable molecule may consist of any compound or substance that is detectable by spectroscopic, photochemical, biochemical, immunochemical or chemical means. For example, useful detectable molecules include radioactive substance (including those comprising ³²P, ³⁵S, ³H, or ¹²⁵I), fluorescent dyes (including 5-bromodesoxyrudin, fluorescein, acetylaminofluorene or digoxigenin), fluorescent proteins (including GFPs and YFPs), or detectable proteins or peptides (including biotin, polyhistidine tails or other antigen tags like the HA antigen, the FLAG antigen, the c-myc antigen and the DNP antigen). According to the invention, the detectable molecule is located at, or bound to, an amino acid residue located outside the said amino acid sequence of interest, in order to minimise or prevent any artefact for the binding between said polypeptides or between the candidate compound and or any of said polypeptides.

In another particular embodiment, the polypeptides are fused with a GST tag (Glutathione S-transferase). In this embodiment, the GST moiety of the said fusion protein may be used as detectable molecule. In the said fusion protein, the GST may be located at the N-terminal end. The GST detectable molecule may be detected when it is subsequently brought into contact with an anti-GST antibody, including with a labelled anti-GST antibody. Anti-GST antibodies labelled with various detectable molecules are easily commercially available.

In another particular embodiment, the nucleolin or the RGG domain of nucleolin or EF-Tupolypeptides are fused with a poly-histidine tag. In a further embodiment, the polypeptides are fused with a protein moiety consisting of either the DNA binding domain or the activator domain of a transcription factor. Said protein moiety domain of transcription may be located either at the N-terminal end or at the C-terminal end. Such a DNA binding domain may consist of the well-known DNA binding domain of LexA protein originating form E. Coli. Moreover said activator domain of a transcription factor may consist of the activator domain of the well-known Gal4 protein originating from yeast.

In a particular embodiment of the screening method according to the invention, the nucleolin or the RGG domain of nucleolin and the EF-Tu are labelled with a fluorescent molecule or substrate. Therefore, the potential alteration effect of the candidate compound to be tested on the binding between the nucleolin or the RGG domain of nucleolin and EF-Tu is determined by fluorescence quantification.

For example, nucleolin or the RGG domain of nucleolin and EF-Tu as above defined may be fused with auto-fluorescent polypeptides, as GFP or YFPs. Nucleolin or the RGG domain of nucleolin and EF-Tu may also be labelled with fluorescent molecules that are suitable for performing fluorescence detection and/or quantification for the binding between said polypeptides using fluorescence energy transfer (FRET) assay. Nucleolin or the RGG domain of nucleolin and EF-Tu may be directly labelled with fluorescent molecules, by covalent chemical linkage with the fluorescent molecule as GFP or YFP. Nucleolin or the RGG domain of nucleolin and EF-Tu may also be indirectly labelled with fluorescent molecules, for example, by non covalent linkage between said polypeptides and said fluorescent molecule. Actually, nucleolin or the RGG domain of nucleolin and EF-Tu may be fused with a receptor or ligand and said fluorescent molecule may be fused with the corresponding ligand or receptor, so.that the fluorescent molecule can non-covalently bind to nucleolin or the RGG domain of nucleolin and EF-Tu. A suitable receptor/ligand couple may be the biotin/streptavidin paired member or may be selected among an antigen/antibody paired member. For example, a polypeptide according to the invention may be fused to a poly-histidine tail and the fluorescent molecule may be fused with an antibody directed against the poly-histidine tail.

As already specified, the screening method according to the invention encompasses determination of the ability of the candidate compound to inhibit the interaction between nucleolin or the RGG domain of nucleolin and EF-Tu by fluorescence assays using FRET. Thus, in a particular embodiment, nucleolin or the RGG domain of nucleolin is labelled with a first fluorophore substance and EF-Tu is labelled with a second fluorophore substance. The first fluorophore substance may have a wavelength value that is substantially equal to the excitation wavelength value of the second fluorophore, whereby the bind of said first and second polypeptides is detected by measuring the fluorescence signal intensity emitted at the emission wavelength of the second fluorophore substance. Alternatively, the second fluorophore substance may also have an emission wavelength value of the first fluorophore, whereby the binding is detected by measuring the fluorescence signal intensity emitted at the wavelength of the first fluorophore substance.

The fluorophores used may be of various suitable kinds, such as the well-known lanthanide chelates. These chelates have been described as having chemical stability, long-lived fluorescence (greater than 0,1 ms lifetime) after bioconjugation and significant energy-transfer in specificity bioaffinity assay. Document US 5,162,508 discloses bipyridine cryptates. Polycarboxylate chelators with TEKES type photosensitizers (EP0203047A1) and terpyridine type photosensitizers (EP0649020A1) are known. Document WO96/00901 discloses diethylenetriaminepentaacetic acid (DPTA) chelates which used carbostyril as sensitizer. Additional DPT chelates with other sensitizer and other tracer metal are known for diagnostic or imaging uses (e.g., EP0450742A1).

In a preferred embodiment, the fluorescence assay consists of a Homogeneous Time Resolved Fluorescence (HTRF) assay, such as described in document WO 00/01663 or US6,740,756, the entire content of both documents being herein incorporated by reference. HTRF is a TR-FRET based technology that uses the principles of both TRF (time-resolved fluorescence) and FRET. More specifically, the one skilled in the art may use a HTRF assay based on the time-resolved amplified cryptate emission (TRACE) technology as described in Leblanc *et al.* (2002). The HTRF donor fluorophore is Europium Cryptate, which has the long-lived emissions of lanthanides coupled with the stability of cryptate encapsulation. XL665, a modified allophycocyanin purified from red algae, is the HTRF primary acceptor fluorophore. When these two fluorophores are brought together by a biomolecular interaction, a portion of the energy captured by the Cryptate during excitation is released through fluorescence emission at 620nm, while the remaining energy is transfered to XL665. This energy is then released by XL665 as specific fluorescence at 665 nm. Light at 665nm is emitted only through FRET with Europium. Because Europium Cryptate is always present in the assay, light at 620nm is detected even when the biomolecular interaction does not bring XL665 within close proximity.

Therefore in one embodiment, the screening method may therefore comprises the steps consisting of:
(1) bringing into contact a pre-assay sample comprising :
   - nucleolin or the RGG domain of nucleolin fused to a first antigen,
   - EF-Tu fused to a second antigen
   - a candidate compound to be tested
(2) adding to the said pre assay sample of step (2) :
   - at least one antibody labelled with a European Cryptate which is specifically directed against the first said antigen
   - at least one antibody labelled with XL665 directed against the second said antigen
(3) illuminating the assay sample of step (2) at the excitation wavelength of the said European Cryptate
(4) detecting and/or quantifying the fluorescence signal emitted at the XL665 emission wavelength
(5) comparing the fluorescence signal obtained at step (4) to the fluorescence obtained wherein pre assay sample of step (1) is prepared in the absence of the candidate compound to be tested.

If at step (5) as above described, the intensity value of the fluorescence signal is lower than the intensity value of the fluorescence signal found when pre assay sample of step (1) is prepared in the absence of the candidate compound to be tested, then the candidate compound may be positively selected at step b) of the screening method.

Antibodies labelled with a European Cryptate or labelled with XL665 can be directed against different antigens of interest including GST, poly-histidine tail, DNP, c-myx, HA antigen and FLAG. Such antibodies encompass those which are commercially available from CisBio (Bedfors, MA, USA), and notably those referred to as 61 GSTKLA or 61HISKLB respectively.

### Two-hybrid assay:

In one embodiment of the screening method according to the invention, each of nucleolin or the RGD domain of nucleolin and EF-Tu comprises a portion of a transcription factor. In said assay, the interaction of nucleolin or the RGD domain of nucleolin and EF-Tugenerates a functional transcription factor that binds to a specific regulatory DNA sequence, which in turn induces expression of a reporter DNA sequence, said expression being further detected and/or measured. A positive detection of the expression of said reporter DNA sequence means that an active transcription factor is formed, due to interaction of nucleolin or the RGD domain of nucleolin and EF-Tu.

Usually, in a two-hybrid assay, the first and second portions of a transcription factor consist respectively of (i) the DNA binding domain of a transcription factor and (ii) the activator domain of a transcription factor. In some embodiments, the DNA binding domain and the activator domain both originate from the same naturally occurring transcription factor. In some embodiments, the DNA binding domain and the activator domain originate from distinct naturally occurring factors, while, when bound together, these two portions form an active transcription factor. The term "portion" when used herein for transcription factor, encompasses complete proteins involved in multi protein transcription factors, as well as specific functional protein domains of a complete transcription factor protein.

Therefore in one embodiment of the invention, the screening method of the invention comprises the following steps:
(1) providing a host cell expressing :
   - a first fusion polypeptide between (i) nucleolin or the RGD domain of nucleolin and (ii) a first protein portion of transcription factor
   - a second fusion polypeptide between (i) EF-Tu and (ii) a second portion of a transcription factor
   said transcription factor being active on DNA target regulatory sequence when the first and second protein portion are bound together and
   said host cell also containing a nucleic acid comprising (i) a regulatory DNA sequence that may be activated by said active transcription factor and (ii) a DNA report sequence that is operatively linked to said regulatory sequence
(2) bringing said host cell provided at step 1) into contact with a candidate compound to be tested
(3) determining the expression level of said DNA reporter sequence.

The expression level of said DNA reporter sequence that is determined at step (3) above is compared with the expression of said DNA reporter sequence when step (2) is omitted. A lower expression level of said DNA reporter sequence in the presence of the candidate compound means that the said candidate compound effectively inhibits the interaction of nucleolin or the RGG domain of nucleolin and EF-Tu and that said candidate compound may be positively selected a step b) of the screening method.
Similar systems of two-hybrid assays are well known in the art and therefore can be used to perform the screening method according to the invention (see. Fields *et al*. 1989 ; Vasavada *et al*. 1991 ; Fearon *et al*. 1992 ; Dang *et al*., 1991, Chien *et al*. 1991, US 5,283,173, US 5,667,973, US 5,468,614, US 5,525,490 and US 5,637,463). For instance, as described in these documents, the Gal4 activator domain can be used for performing the screening method according to the invention. Gal4 consists of two physically discrete modular domains, one acting as the DNA binding domain, the other one functioning as the transcription-activation domain. The yeast expression system described in the foregoing documents takes advantage of this property. The expression of a Gal1-LacZ reporter gene under the control of a Gal4-activated promoter depends on the reconstitution of Gal4 activity via protein-protein interaction. Colonies containing interacting polypeptides are detected with a chromogenic substrate for β-galactosidase. A complete kit (MATCHMAKER®,) for identifying protein-protein interactions is commercially available from Clontech.

So in one embodiment, nucleolin or the RGD domain of nucleolin is fused to the DNA binding domain of Gal4 and EF-Tu is fused to the activation domain of Gal4.

The expression of said detectable marker gene may be assessed, by quantifying the amount of the corresponding specific mRNA produced. However, usually the detectable marker gene sequence encodes for detectable protein, so that the expression level of the said detectable marker gene is assessed, by quantifying the amount of the corresponding protein produced. Techniques for quantifying the amount of mRNA or protein are well known in the art. For example, the detectable marker gene placed under the control of regulatory sequence may consist of β-galactosidase.

### Western blotting

In another particular embodiment, the screening method comprises subjecting to a gel migration assay the mixture of nucleolin or the RGG domain of nucleolin and EF-tu, with or without the candidate compound to be tested and then measuring the binding of the said polypeptides altogether by performing a detection of the complexes formed between said polypeptides. The gel migration assay can be carried out as known by the one skilled in the art.

Therefore in one embodiment of the invention, the screening method of the invention comprises the following steps :
(1) providing nucleolin or the RGG domain of nucleolin and EF-Tu
(2) bringing into contact the candidate compound to be tested with said polypeptides
(3) performing a gel migration assay a suitable migration substrate with said polypeptides and said candidate compound as obtained at step (2)
(4) detecting and quantifying the complexes formed between said polypeptides on the migration assay as performed at step (3).

The presence or the amount of the complexes formed between the polypeptides is then compared with the results obtained when the assay is performed in the absence of the candidate compound to be tested. Therefore, when no complexes between the polypeptides is detected or, alternatively when those complexes are present in a lower amount compared to the amount obtained in the absence of the candidate compound, then the candidate compound may be positively selected at step b) of the screening method.

The detection of the complexes, formed between the said two polypeptides ,may be easily performed by staining the migration gel with a suitable dye and then determining the protein bands corresponding to the protein analysed since the complexes formed between the first and the second polypeptides possess a specific apparent molecular weight. Staining of proteins in gels may be done using the standard Coomassie brilliant blue (or PAGE blue), Amido Black, or silver stain reagents of different kinds. Suitable gels are well known in the art such as sodium dodecyl (lauryl) sulfate-polyacrylamide gel. In a general manner, western blotting assays are well known in the art and have been widely described (Rybicki *et al*., 1982; Towbin *et al*. 1979; Kurien *et al*. 2006).

In a particular embodiment, the protein bands, corresponding to the polypeptides submitted to the gel migration assay can be detected by specific antibodies. It may use both antibodies directed against nucleolin or the RGG domain of nucleolin and antibodies specifically directed against EF-Tu.

In another embodiment, the two polypeptides are labelled with a detectable antigen as above described. Therefore, the protein bands can be detected, by specific antibodies directed against said detectable antigen. Preferably, the detectable antigen conjugates to nucleolin or the RGG domain of nucleolin is different from the antigen conjugated to EF-Tu. For instance, nucleolin or the RGG domain of nucleolin can be fused to a GST detectable antigen and EF-Tu can be fused with the HA antigen. Then the protein complexes formed between the two polypeptides may be quantified and determined with antibodies directed against the GST and HA antigens respectively.

### Biosensor assays

In another embodiment, the screening method comprises using an optical biosensor such as described by Edwards *et al*. (1997) or also by Szabo *et al.* (1995). This technique allows the detection of interactions between molecules in real time, without the need of labelled molecules. This technique is indeed based on the surface plasmon resonance (SPR) phenomenon. Briefly, a first protein partner is attached to a surface (such as a carboxymethyl dextran matrix). Then the second protein partner is incubated with the previously immobilised first partner, in the presence or absence of the candidate compound to be tested. Then the binding including the binding level, or the absence of binding between said protein partners is detected. For this purpose, a light beam is directed towards the side of the surface area of the substrate that does not contain the sample to be tested and is reflected by said surface. The SPR phenomenon causes a decrease in the intensity of the reflected light with a combination of angle and wavelength. The binding of the first and second protein partner causes a change in the refraction index on the substrate surface, which change is detected as a change in the SPR signal.

It is preferable to use the RGD domain of nucleolin rather than full nucleolin in this assay.

### Affinity chromatography

In another one embodiment of the invention, the screening method includes the use of affinity chromatography.

Candidate compounds can be selected by any immunoaffinity chromatography technique using any chromatographic substrate onto which (i) nucleolin or the RGG domain of nucleolin or (ii) EF-Tu as above defined, has previously been immobilised, according to techniques well known from the one skilled in the art. Briefly, nucleolin or the RGG domain of nucleolin or EF-Tu, may be attached to a column using conventional techniques including chemical coupling to a suitable column matrix such as agarose, Affi Gel®, or other matrices familiar to those of skill in the art. In some embodiment of this method, the affinity column contains chimeric proteins in which the nucleolin or the RGG domain of nucleolin or EF-Tu, is fused to glutathion-s-transferase (GST). Then a candidate compound is brought into contact with the chromatographic substrate of the affinity column previously, simultaneously or subsequently to the other polypeptide among the first and second polypeptide. After washing, the chromatography substrate is eluted and the collected elution liquid is analysed by detection and/or quantification of the later applied first or second polypeptide, so as to determine if, and/or to which extent, the candidate compound has impaired the interaction of nucleolin or the RGG domain of nucleolin and EF-Tu.

### Candidate compounds :

According to a one embodiment of the invention, the candidate compound may be selected from a library of compounds previously synthesised, or a library of compounds for which the structure is determined in a database, or from a library of compounds that have been synthesised de novo.

The candidate compound may be selected from the group of (a) proteins or peptides, (b) nucleic acids and (c) organic or chemical compounds. Illustratively, libraries of pre-selected candidate nucleic acids may be obtained by performing the SELEX method as described in documents US 5,475,096 and US 5,270,163. Further illustratively, the candidate compound may be selected from the group of antibodies directed against nucleolin or the RGG domain of nucleolin and EF-Tu.

### In cellulo screening methods :

The candidate compounds that have been positively selected at the end of any one of the embodiments of the in vitro screening which has been described previously in the present specification may be subjected to further selection steps in view of further assaying its anti-Tularemia biological properties. For this purpose, the candidate compounds that have been positively selected with the general in vitro screening method as above described may be further selected for their ability to inhibit the formation of infectious Tularemia infected cells.

The invention will be further illustrated through the following examples, figures and tables.

### LEGENDS TO THE FIGURES :

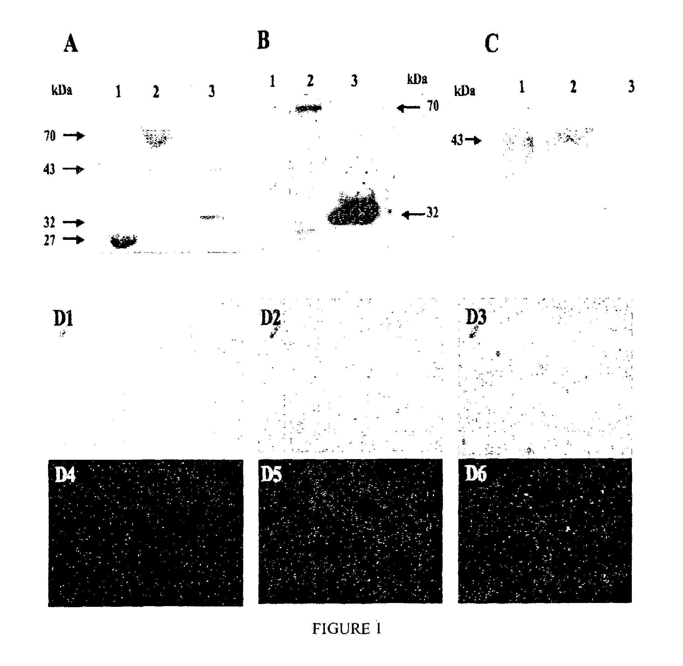

**Figure 1****.** Elongation factor EF-Tu is localized at the surface of *F. tularensis* LVS. **A** and **B:** SDS-PAGE of GST (lanes 1), GST-EF-Tu (lanes 2) and His-EF-Tu (lanes 3) either stained with Coomassie Blue (A) or immunoblotted with anti-EF-Tu diluted 1/100,000 (B). **C:** SDS-PAGE of LVS subcellular fractions from 105,000 x g supernatant (lane 1), proteins solubilized by NP40 from membrane fractions (lane 2) or not solubilized (lane 3), immunoblotted with anti-EF-Tu diluted 1/5,000. In A, B and C, the estimated molecular mass (in kDa) of each protein is indicated on the left of the black lines. **D:** Microscopy analysis of a pellet of LVS bacteria incubated either with pre-immune serum diluted 1/200 (D1, D4), polyclonal anti-LVS diluted 1/10,000 (D2, D5), or anti-EF-Tu Ab diluted 1/200 (D3, D6). Bright fields (D1, D2, D3) or fluorescence microscopy at a 63x magnification (D4, D5, D6) of 10 fields were analyzed for each group. These images are representative of 3 different experiments.

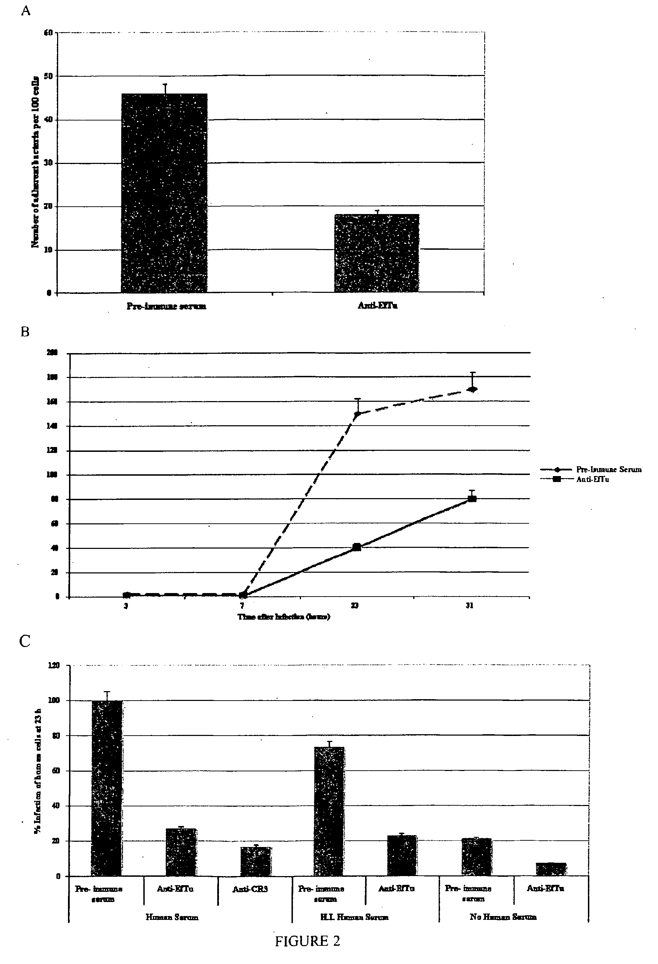

**Figure 2****.** Elongation factor Tu of LVS participates in binding of LVS to human monocytes. **A**: LVS bacteria transformed with a GFP expressing plasmid (LVS-GFP), first incubated either with pre-immune serum or with anti-EF-Tu (diluted 1/200), were added to monocytic THP-1 human cells in suspension (M.O.I. 300:1) and analyzed by fluorescence microscopy at 63x magnification. Fifteen fields containing an average of 100 cells were examined to quantify the number of bacteria adherent on human cells (p = 4.9 x10⁻⁹, as calculated by Student's *t* test). **B:** Kinetics of LVS infection. LVS bacteria were pre-incubated with human serum AB containing intact complement activity. Then, LVS was incubated either with rabbit pre-immune serum (dashed line) or anti-EF-Tu (plain line), both diluted 1/200. Human monocytes were then infected at M.O.I. 200:1, for different times at 37°C. For quantification of bacteria multiplication inside the cells, an aliquot of THP-1 cells was removed at indicated times, serially diluted and the CFU were counted by plating on chocolate agar. Each point was performed in triplicate and this experiment was representative of 5 different experiments (p = 0.025 and 0.039 for 23 h and 31 h, respectively, as determined by Student's *t* test). **C:** LVS bacteria were incubated with Human Serum with intact complement activity, H.I. Human Serum or in absence of human serum. LVS bacteria in these different conditions were further incubated either with rabbit pre-immune serum or anti-EF-Tu, both diluted 1/200. Human monocytes were then infected at M.O.I. 200:1. A sample of monocytes, infected with opsonized LVS, was also pre-incubated with anti-CR3 MAb. Cells were then washed with RPMI with gentamicin and further incubated in RPMI with 5% FCS and gentamicin for 23 h. An aliquot was taken for quantifying bacteria multiplication inside the cells. Each point was performed in triplicate and this experiment is representative of 5 different experiments (p = 8 x10⁻⁵ for conditions without serum, as determined by Student's *t* test).

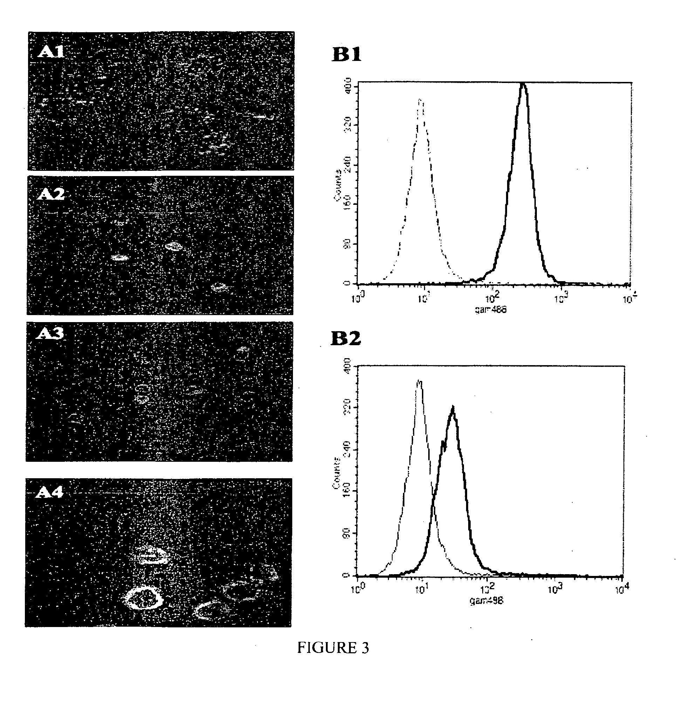

**Figure 3****.** Presence of nucleolin on monocytic cell surface correlates with state of infection. **A:** THP-1 cells were incubated with either anti-nucleolin MAb, diluted 1/200 followed by Alexa Fluor 488-labeled GAM (A1 and A2) or Alexa Fluor 563-labeled phalloidin (A3). Cells were fixed with PFA. Permeabilization by Triton X100 was performed only in A2 and A3. A sample of THP-1 cells was incubated first with cytochalasin D (A4) and treated as A1. Analysis was performed by fluorescence microscopy at 100x magnification (A1 and A4) or X 63 (A2 and A3) magnification. **B:** FACS analysis of nucleolin expression on cell surface of uninfected cells (B1) or cells infected by LVS (M.O.I. 1:200) (B2). Cells were then incubated either with anti-GAPDH as isotype negative control (dotted line) or with anti-nucleolin MAb (plain line). Binding of MAbs on cell surface was followed by incubation with Alexa Fluor 488-labeled GAM. For each sample, 10,000 viable cells were gated and fluorescence intensity was analyzed with Cell Quest ^{™} Software.

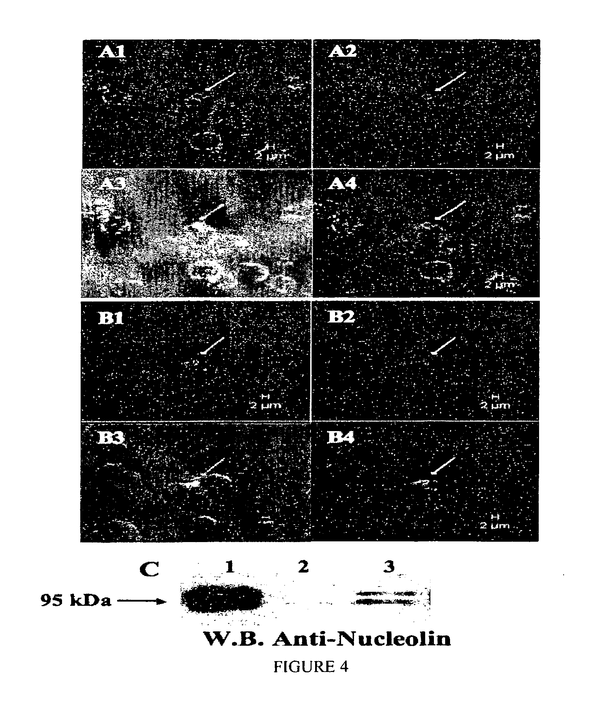

**Figure 4****.** Cell surface nucleolin interacts with LVS elongation factor Tu. **A** and **B:** THP-1 cells were incubated with opsonized LVS (M.O.I. 200:1) and their interaction was observed by confocal microscopy at X 63 magnification. Human cell surface was labeled with anti-nucleolin MAb, detected by Alexa Fluor 488-labeled GAM (A1 and B1). Bacteria were labeled either with polyclonal anti-LVS, diluted 1/10,000 (A2) or anti-EF-Tu diluted 1/2,000 (B2) detected by Alexa Fluor 546-labeled GAR. Merging was observed either with fluorescence light (A4, B4) or bright field (A3, B3). Arrows indicate the LVS bacteria bound on cell surface and already engulfed by cell membrane. This experiment is representative of five different experiments. C: GST (lane 2) or GST-EF-Tu (lane .3) bound on glutathione-Sepharose beads were incubated with THP-1 cell membrane proteins. After extensive washes, bound proteins were eluted with sample buffer with reducing conditions and run on SDS-PAGE with, in control, proteins solubilized from THP-1 cell membranes, run directly without incubation with beads (lane 1). Immunoblotting experiment was performed with anti-nucleolin MAb. The estimated molecular mass (in kDa) of nucleolin is indicated on the left of the black line.

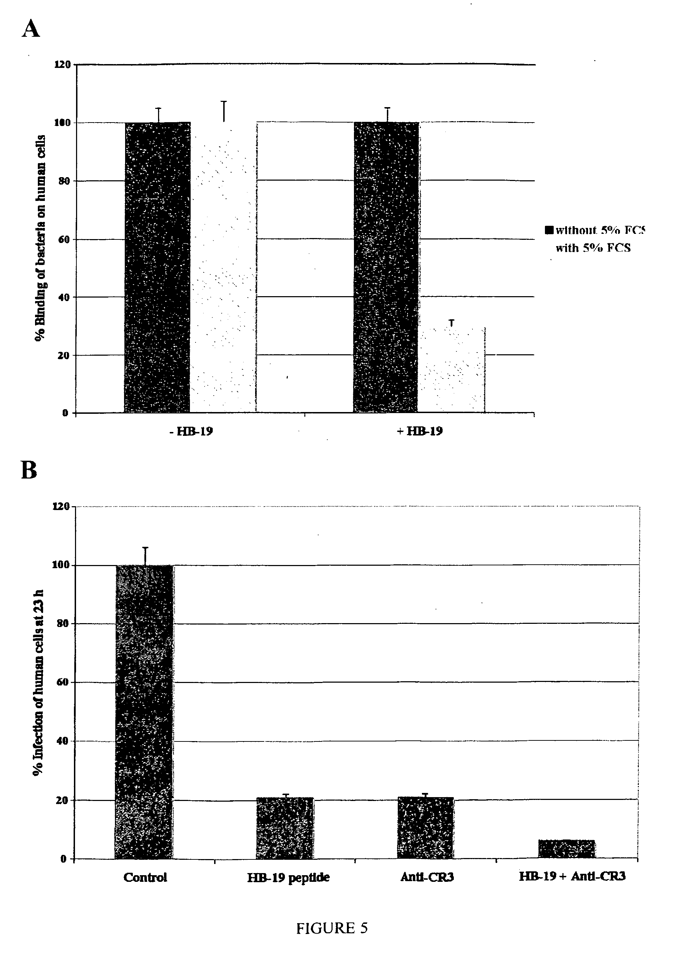

**Figure 5****.** Interaction of nucleolin with LVS EF-Tu is involved in binding and infection of human monocytes. **A:** Human monocytes in suspension, in presence or absence of 5% FCS, were incubated with or without 3 µM HB-19 pseudopeptide. LVS-GFP bacteria were then added at a M.O.I. 300:1. Cells were washed in RPMI and analyzed by fluorescence microscopy. Twenty-five fields containing an average of 100 cells were examined to quantify the number of bacteria adherent on human cells (p = 1.6 x10⁻¹⁶, as determined by Student's *t* test). **B:** Human monocytes, in RPMI with 5% FCS were incubated in presence of anti-GAPDH MAb used as control, anti-CR3 receptor MAb or 3 µM HB-19 pseudopeptide alone or together with anti-CR3. Then, opsonized LVS bacteria were added at a M.O.I. 200:1. Cells were washed with RPMI containing gentamicin and further incubated in RPMI with 5% FCS and gentamicin for 23 h at 37°C. An aliquot was then taken for quantifying bacteria multiplication inside the cells. Each point was performed in triplicate and this experiment was representative of 5 different experiments (p = 4.4 x10⁻⁴ for cells incubated either with anti-CR3 alone or with HB-19, as determined by Student's *t* test).

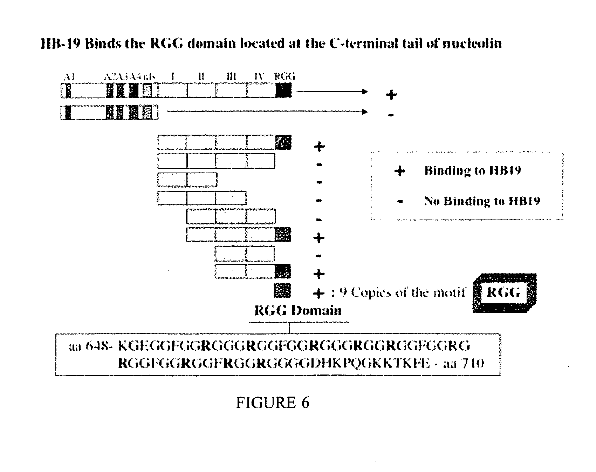

**Figure 6****.** The structure of human nucleolin and deletion constructs. The figure shows a schematic structure of nucleolin (amino acids 1-710) and the constructs corresponding to the N-terminal portion (amino acids 1-308) and C-terminal portion (amino acids 309-710) of nucleolin. The positions of the long stretches of acidic domains (A1, A2, A3, and A4), the bi-partite nuclear localization signal (nls), the four RNA binding domains I, II, III, and IV, and the C-terminal tail containing the nine repeats of RGG are as indicated. The deletion constructs of the C-terminal part of nucleolin (amino acids 309-710) were generated as a fusion protein with GST; GST is at the N-terminal end of the fusion protein. Eight deletion constructs were generated expressing different RBDs with or without the RGG domain. The + and - signs next to each construct indicates binding capacity to HB-19.

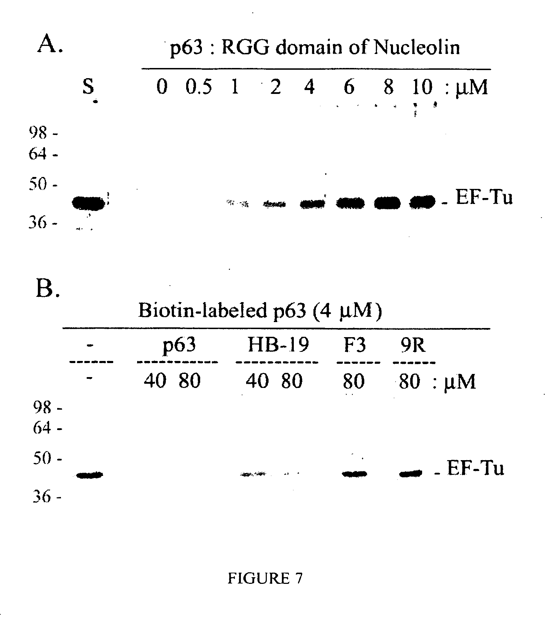

**Figure 7****.** F. tularensis EF-Tu binds the C-terminal RGG domain of nucleolin. **A.** The p63 peptide corresponding to the RGG domain of nucleolin binds in a dose dependent manner recombinant EF-Tu. Aliquots containing 1µg of the purified recombinant His-EF-Tu (as described in Fig. 1) in 100 µl of PBS were incubated (4°C, 2 h) with different concentrations of the biotinylated p63 peptide (0, 0.5, 1, 2, 4, 6, 8, 10 µM). The complex formed between His-EF-Tu and the biotinylated p63 peptide was isolated by purification using avidin-agarose (100 µl; ImmunoPure Immobilized Avidin from Pierce). After 2 h of incubation at 4 °C, the samples were washed extensively with PBS containing 1 mM EDTA. The purified proteins were denatured by heating in the electrophoresis sample buffer containing SDS and analyzed by SDS-PAGE. Lane S shows the purified preparation of EF-Tu (material corresponding to 0.2 µg protein). The presence of EF-Tu was then revealed by immunoblotting using the rabbit anti-EF-Tu antibody (at 50,000-fold dilution). **B.** The specific binding of EF-Tu with the RGG domain of nucleolin. Aliquots containing 1 mg of His-EF-Tu in 100 µl of PBS were preincubated (4°C, 45 min) as such (lane -), or with 40 and 80 µM unlabeled p63 peptide (panel p63, 40 and 80), or with 40 and 80 µM HB-19 (panel HB-19, 40 and 80), or with 80 µM F3 peptide (panel F3, lane 80), or with 80 µM 9Arg peptide (panel 9A, lane 80) before further incubation (4°C, 2 h) with the biotinylated p63 peptide (4 µM). The complex formed between His-EF-Tu and the biotinylated p63 peptide was isolated and analyzed by immunoblotting as in section A. On the left is the position of molecular weight protein markers of 98, 64, 50, and 36 kDa. On the right is the position of the 43 kDa His-EF-Tu.
**Figure 8****:**
   **A.** Structure of HB-19;
   **B.** Structure of Nucant 01 which comprises a cyclic hexapeptide as a template, constituted of an alternance of alanine (A) having D configuration and lysine (K) having L configuration; Three pseudopeptide motifs KΨPR (wherein Ψ = CH₂-N) are covalently linked to the ε amino group of each of the 2 lysine residues;
   **C.** Structure of Nucant 2 which comprises a linear peptide that can show an helix, as a template, on which 5 pseudopeptide motifs KΨPR (wherein Ψ = CH₂-N) are covalently linked to the ε amino group of each of the 5 lysine residues, Ac representing CH₃-CO-;
   **D.** Structure of Nucant 3 which comprises a linear peptide that can show an helix, as a template, on which 5 pseudopeptide motifs KΨPR (wherein Ψ = CH₂-N) are covalently linked to the ε amino group of each of the 5 lysine residues, Ac representing CH₃-CO-;
   **E.** Structure of Nucant 6 which comprises a linear peptide that can show an helix, as a template, on which 6 pseudopeptide motifs KΨPR (wherein Ψ = CH₂-N) are covalently linked to the ε amino group of each of the 6 lysine residues, Ac representing CH₃-CO-;
   **F.** Structure of Nucant 7 which comprises a linear peptide that can show an helix, as a template, on which 6 pseudopeptide motifs KΨPR (wherein Ψ = CH₂-N) are covalently linked to the ε amino group of each of the 6 lysine residues, Ac representing CH₃-CO-.

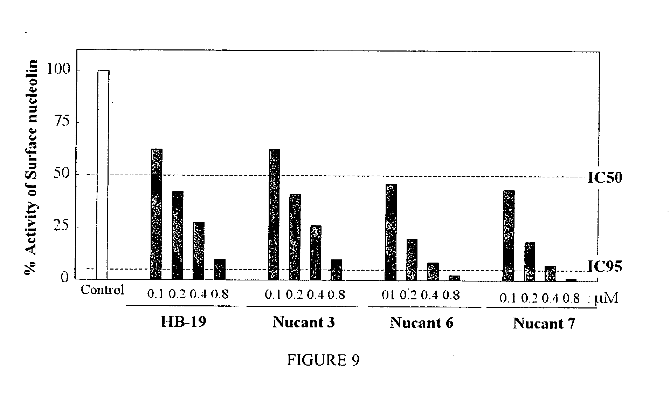

**Figure 9****.** Activity of nucleolin on the surface of cells treated with different concentrations of HB-19, Nucant 3, Nucant 6, and Nucant 7.

### EXAMPLES

### Materials and methods

### Bacteria, mammalian cells, antibodies and human serum

Live vaccine strain, *F. tularensis* LVS, was kindly supplied by A. Sjöstedt and grown either in Schaedler broth containing vitamin K3 or on chocolate agar plates containing polyvitex (Biomerieux) at 37°C. LVS-GFP was obtained by transformation of LVS bacteria with pFNLTP6 *gro-gfp,* kindly provided by Dr. Zahrt (Maier, T. M., 2004). The human monocyte-like cell line THP-1 was grown in suspension in RPMI containing 10% (v/v) FCS at 37°C in a CO₂ incubator. Antibodies (Abs) used were: polyclonal rabbit anti-EF-Tu Ab prepared in our laboratory, anti-LVS Ab (Becton Dickinson), anti-GST (Oncogene) or rabbit pre-immune serum; MAbs were anti-nucleolin Ab (clone D3) (Deng, H., 1996) , anti-glyceraldehyde phosphate dehydrogenase (GAPDH) (Santa Cruz Biotechnology) as an isotype-matched irrelevant control for nucleolin and anti-CD11/CD18 (anti-CR3 receptors) (Abcam), which specifically recognizes the human CD11/18 activation epitope. Secondary antibodies were GAR or GAM conjugated to HRP (Dako) for immunoblotting experiments or Alexa Fluor cytochromes for microscopy experiments. Human serum AB (PAA) was handled in a manner to preserve complement activity (Sanchez-Corral, P., 1989). Heat-inactivated (H.I.) serum was obtained by incubating serum for 30 min at 56°C. HB-19 pseudopeptide was synthesized as previously described (Nisole, S., 1999).

### Preparation of subcellular fractions of LVS

After 24 h of culture, LVS were collected by centrifugation and suspended in 50 mM Tris/HCl pH 8.0. Bacteria were disintegrated with Fast-Prep beads using 4 repeated cycles of 30 sec at a 6.5 speed. After 10 min on ice, undisrupted microbes were eliminated by centrifugation for 5 min at 2,800 x g. Supernatant was then centrifuged for 60 min at 105,000 x g in a Beckmann Optima Max ultracentrifuge, at 4°C. The pellet, which contained the bacterial membranes was solubilized for 45 min at 4°C in TNE (50 mM Tris, 150 mM NaCl, 1 mM EDTA, pH 8.0 containing 1% NP40 and 1% Triton X100). A further centrifugation at 12,000 x g for 20 min at 4°C separated the solubilized membrane proteins present in the supernatant from membrane-associated proteins present in the pellet.

### Preparation of EF-Tu recombinant proteins and production of polyclonal antibodies

Chromosomal DNA was purified from LVS with Turbogen kit (Invitrogen). PCR reactions were performed in Bio-Rad I Thermal Cycler with 100 pmoles/µl of 5' primer 5'-CCGGAATTCATGGCTAAAGAAAAATTTGAGCGTTC -3' (nt 3141 to 3156) SEQ ID NO: 18
and 3' primer was
5'-CGAGT*CGAC*TTACTCGATAATTTTAGCTACAACACCAGCACC-3' (nt 3246 to 3261). SEQ ID NO:19.

The EF-Tu amplicon of 1.1 kb was purified on agarose gel, cloned in pCR2-TOPO1 vector (Invitrogen) and sequenced in Institut Cochin (Genomic Sequencing Department). Once its sequence confirmed, EF-Tu was subcloned in pGEX-4T1 (Amersham Biosciences) or pET28a+ (Novagen) into *Eco*RI-*Sal*I sites, in DH5α. After transforming in *E. coli* strain BL21 or BL21 DE3, respectively, GST-fusion proteins and His-fusion proteins were produced as previously described (Balbo, M., 2005) and purified either on glutathione-Sepharose 4B beads (Amersham Biosciences) or on Ni-column (Novagen), as described by manufacturers. His-fusion EF-Tu protein was used as immunogen for immunizing rabbits.

### Pull-down assay and Immunoblotting assays

Pull-down assay was performed by incubating 5 µg of GST or GST-EF-Tu bound on glutathione-Sepharose beads with 500 µg proteins solubilized from THP-1 cell membranes by NP40 in Phosphate-buffered saline (PBS) containing protease inhibitors (Roche Diagnostics), as previously described (Barel, M., 2001). After washes of beads, bound proteins were eluted in sample buffer and submitted to immunoblotting assays. Recombinant proteins, or proteins present in the different fractions obtained after subcellular fractionation of LVS, were submitted to SDS-PAGE. Separated proteins were either directly stained with Coomassie Blue or electrotransferred on nitrocellulose sheet (Schleicher & Schuell), using a semi-dry Transblot apparatus (Bio-Rad). Membranes were incubated in PBS containing 5% powdered milk and then with the indicated Abs. After washing in PBS/0.05% Tween-20 (PBS-T), peroxidase-linked secondary Abs were added. Nitrocellulose sheets were washed and binding of second Abs was detected using the ECL kit (Amersham).

### Infection of mammalian cells

Thirty minutes before infection, LVS bacteria were suspended at 37°C in RPMI alone or containing 10% human serum or H.I. serum. Human monocytic cells were washed in RPMI and suspended in RPMI with or without 5% FCS. In selected experiments, monocytes were pretreated with cytochalasin D (Sigma), an actin polymerization inhibitor or with 3· µM HB-19 pseudopeptide (Nisole, S., 1999). Then, human cells were diluted with untreated or treated LVS and incubated at 37°C for 30 min. Cells were washed in RPMI with gentamicin and further incubated for different times at 37°C in RPMI containing 5% FCS and gentamicin. The number of intracellular bacteria present in monocytes at different times was determined by plating serial dilutions of THP-1 cells lysed in distilled water on chocolate agar plates, followed by enumeration of bacteria.

### Fluorescent and Confocal experiments

Infected or uninfected THP-1 monocyte cells were first washed in PBS / Ca²⁺Mg²⁺ (Gibco) and suspended at 2x10⁶ cells/ml. Then, cells were prepared either for extracellular or intracellular labeling. For extracellular labeling, cells were first incubated with 5% goat serum in PBS, to block non-specific binding sites, then with first Abs. After washing, cells were incubated with second Abs (Alexa Fluor 488-labeled GAM and/or Alexa Fluor 546-labeled GAR) in the dark. After washing, cells were fixed with para-formaldehyde (PFA) and incubated with 50 mM NH₄Cl to quench residual aldehydes. After centrifugation, the cell pellet was suspended in Mowiol and mounted on glass coverslips for analysis by either fluorescence or confocal microscopy. For intracellular labeling, cells were first fixed as described above with PFA before being permeabilized by Triton X-100. After incubation in NH₄Cl, cells were centrifuged, and then submitted to the same protocol as described above for extracellular labeling. Incubation with Alexa Fluor 563-labeled phalloidin was used for detecting integrity of the cell cytoskeleton. After labeling with second Abs and washing, cells were pelleted. The pellets were suspended in Mowiol as described above. For binding experiments with LVS-GFP, cells were treated as described above for external labeling, without incubation with second Ab. Cells were visualized with a fluorescence microscope (Zeiss Axioplan 2) using Qimaging Digital Camera with Q Capture Pro Fluorescence analysis software or at the appropriate wavelengths under a Zeiss LSM 5 Pascal confocal microscope with argon (458/488 nm) and helium neon (543 nm) lasers with LSM analysis software. We inspected more than 500 cells per experiment and present the photographs of cells with typical morphology and staining.

Labeling and fixation of LVS bacteria was performed as described above for extracellular labeling of human cells.

### FACS analysis

THP-1 monocytic cells uninfected or infected by LVS were incubated with anti-nucleolin or anti-GAPDH MAbs. After washing, cells were incubated with Alexa Fluor 488-labeled GAM in dark place. After washing, cells were fixed with PFA and incubated in NH₄Cl. Cells were centrifuged and processed for analysis by FACS scan flow cytometer (Becton Dickinson). For each sample, 10,000 viable cells were gated following size (forward scatter, FSC) and granularity (side scatter (SSC) and analyzed with Cell Quest ^{™} Software (Becton Dickinson).

### Peptide constructs

- The synthesis of 5[Lys (CH2N)Pro-Arg]-template-assembled synthetic peptide referred to as HB-19 was as described in Nisole et al, 1999.
- The nine-D-arginine peptide construct (referred to as 9Arg) was synthesized using Fmoc-protected D-Arg residue (Said et al, 2002). The p63 peptide was synthesized according to the last 63 amino acid residues of human nucleolin (KGEGGFGGRGGGRGGFGGRGGGRGGRGGFGGRGRGGFGGRGGFRGGRGGG GDHKPQGKKTKFE) (SEQ ID N°17) (Nisole et al, 2002).
- The synthetic F3 peptide is a tumor-homing peptide corresponding to the 34 amino acid fragment (AKVKDEPQRRSARLSAKPAPPKPEPKPKKAPAKK, SEQ ID N°20) known to bind the N-terminal end of nucleolin.

For the biotin-labeled p63 peptide, the biotin moiety was introduced during peptide assembly as an Fmoc Lys-biotin derivative at the N-terminus of the peptide. All peptides were obtained at a high purity (95%), and their integrity was controlled by matrix-associated laser desorption ionization-time-of-flight analysis.
- Nucant 01 was synthesized by covalently coupling KΨPR on a cyclic backbone of C3 symetry. The synthesis of the backbone was described in Fournel et al, 2005. The protected Fmoc KΨPR motif was assembled on a chlorotrityl resin then clived from the resin under weakly acidic conditions.

The protected KΨPR motif was then coupled to epsilon NH2 function of each lysine residue (K) of the backbone in a stoechiometry of 1,1 KΨPR/1 cyclic molecule. Coupling was achieved by the activation process BOP/HoBt during 48H.

At the end of the reaction, protecting groups were removed to trifluoroacetic acid and the final compound was precipitated using ether.

Nucant 01 so obtained was purified by HPLC and characterized by mass spectrometry.
- Nucant 2 and Nucant 3 were synthesized as follows: peptide templates known to adopt a helix structure, were assembled on a solid phase using a Boc chemistry. These templates are built from 5 repetitions of Aib-Lys-Aib-Gly (NUCANT ) or Lys-Aib-Gly (NUCANT 3), Aib being 2-amino-isobutyric acid.

The Fmoc protective groups of Lys lateral chain were then removed by pipéridine (3 times 5 minutes) in DMF. KΨPR motifs (wherein Y = CH2-N) were then anchored to the five Lys εNH₂ groups.

The final acidic clivage was performed in hydrofluoric acid.

After precipitation in ether, dissolution under aqueous conditions and lyophilisation, NUCANT 2 andNUCANT 3 were purified by HPLC, analyzed by mass spectrometry and lyophilised.
- Nucant 6 et Nucant 7 were prepared using the same protocol.

### Results :

### EF-Tu is localized at the surface of F. tularensis LVS

The sequence of the LVS gene encoding EF-Tu (FTL_1751) was obtained by *in silico* analysis of LVS genome. LVS EF-Tu presents 99.7 % amino acid identity with *F. tularensis* SCHU S4 EF-Tu (FTT_0137). Purified recombinant EF-Tu, tagged either with a polyhistidine tag (His-EF-Tu) or with glutathione S-transferase protein (GST-EF-Tu), were prepared in *E. coli.* Both recombinant proteins presented the expected apparent molecular mass (MW) on SDS-PAGE (Fig 1A) of 43 kDa for His-EF-Tu and 70 kDa for GST-EF-Tu. An additional product with a MW of 32 kDa, compatible with a proteolysis-sensitive cleavage site in the protein, was always observed in all His-tagged preparations. Notably, a truncated fragment with the same apparent MW has been previously described in other EF-Tu recombinant protein preparations (Archambaud, C., 2005). A polyclonal anti-EF-Tu antibody (anti-EF-Tu Ab) was produced, by immunizing rabbits with His-EF-Tu protein. The specificity of anti-EF-Tu Ab was tested on both GST and His-tagged recombinant proteins (Fig 1B). Anti-EF-Tu Ab specifically recognized GST-EF-Tu but not GST alone and mainly, the 32-kDa cleavage product, suggesting that this 32 kDa product is more immunogenic than the rest of the molecule. Three subcellular fractions of LVS (corresponding to cytosolic, detergent-solubilized membranes and membrane-associated proteins, respectively) were prepared, and their protein contents were tested by Western-blot using anti-EF-Tu Ab (Fig 1C). EF-Tu, with the expected apparent MW of 43 kDa, was detected in the cytosolic fraction (Schilstra, M. J., 1984). EF-Tu was also detected in the fraction containing membrane proteins solubilized by non-ionic detergents, while it was not found in membrane-associated proteins. Presence of EF-Tu at the bacterial surface was detected by fluorescence microscopy (Fig 1D). Incubation of LVS grown in broth with anti-EF-Tu Ab (Fig 1 D6) gave a positive signal of fluorescence. This signal was less diffuse than that obtained when bacteria were incubated with polyclonal anti-LVS Ab (Fig 1D5). As expected, no signal was detected with the pre-immune serum (Fig 1D4). These data indicate that a fraction of EF-Tu is exposed at the surface of LVS.

### EF-Tu of F. tularensis participates in binding of LVS to human monocytes

Involvement of EF-Tu in adhesion of LVS to mammalian monocytic cells was determined by fluorescence analysis, using LVS bacteria expressing green fluorescence protein (GFP) and monocytic THP-1 cells in suspension (Fig 2A). Bacterial cells were pre-incubated either with pre-immune serum or with anti-EF-Tu Ab. The number of adherent fluorescent bacteria (LVS-GFP) was counted on an average number of 100 THP-1 cells, in fifteen different fields. Binding of LVS-GFP to human cells was decreased by 70% when surface-exposed EF-Tu was blocked with anti-EF-Tu Ab, demonstrating that LVS EF-Tu was involved in LVS binding to human monocytes.

The inventors then quantified the kinetics of LVS infection of THP-1 human cells (Fig 2B). Our first experiments were performed in presence of human serum AB, as opsonizatioh of LVS by human complement has been shown to enhance its ability to infect human cells (Clemens, D. L., 2005). The plateau of bacterial multiplication was reached ca. 31 h after infection. After 48 h, cells died, presumably of apoptosis (Lai, X. H., 2001). A strong decrease in the rate of infection was observed when bacteria were pre-incubated with anti-EF-Tu Ab, with the maximum decrease (73%) in bacterial number detected at 23 h. Hence, we used 23 h of infection to compare the level of infection in presence of normal human serum or heat-inactivated serum (H.I. serum) or in absence of serum and to monitor the effect of anti-EF-Tu Ab under these different conditions (Fig 2C). In agreement with earlier observations (Clemens, D. L., 2005 ; Balagopal, A., 2006) , presence of normal serum allowed maximal infection, while presence of heat-inactivated (H.I.) serum or absence of serum induced a lower level of infection (70% and 20%, respectively). When cells were pre-incubated with anti-CR3 Ab, only 18% infection was observed, confirming the involvement of CR3 receptors in uptake of LVS though complement components (Clemens, D. L., 2005 ; Balagopal, A., 2006). Interestingly, the 73% decrease in THP-1 infection quantified when LVS bacteria were pre-incubated with anti-EF-Tu Ab was also observed in absence of complement. This result suggested that this inhibitory effect of anti-EF-Tu Ab on infection was independent of the presence of complement and complement receptors. Altogether, these data support the notions that: i) EF-Tu is involved in LVS binding to- and infection of- human monocytes; ii) this interaction works in addition to that played by the complement-CR3 receptor pair, thus suggesting the involvement of a cellular receptor for EF-Tu.

### Nucleolin interacts with EF-Tu of F. tularensis

The cell surface protein(s) involved in bacterial adhesion, internalization and infection should be able to translocate from cell surface to the cytoplasm. Nucleolin is such a protein. Indeed, nucleolin has been shown to be localized not only in the nucleus, but also on the cell surface and to mediate internalization of specific ligands (Hovanessian, A. G., 2000). This prompted the inventors to evaluate if nucleolin was involved in binding of LVS by interacting with EF-Tu.

The inventors first verified that nucleolin was present on monocytic surface by performing confocal microscopic analysis of cells incubated with a monoclonal antibody (MAb) specific of nucleolin, which was demonstrated to be internalized by an active temperature-dependent process (no internalization occurring at 5°C and very little at room temperature) (Hovanessian, A. G., 2000) (Fig 3A). As reported earlier with other cell lines, when THP-1 cells were not permeabilized, nucleolin was found clustered in patches on cell surface (Fig 3A1). When THP-1 cells were permeabilized, nucleolin was found in the nucleus (Fig 3A2). Labeling with phalloidin demonstrated that permeabilization did not affect the cytoskeleton of the cells (Fig 3A3). Pretreatment of monocytes with cytochalasin D modified the cell-surface expression of nucleolin (Fig 3A4), by triggering the collapse of actin, as has been previously described (Hovanessian, A. G., 2000).

The inventors then looked whether expression of cell surface nucleolin could be modulated by LVS infection (Fig 3B). FACS analysis showed that cell surface expression of nucleolin was considerably decreased when cells were infected by LVS (Fig 3B2), as compared to uninfected cells (Fig 3B1). This result suggested that disappearance of nucleolin from cell surface after bacterial infection could be linked to entry of the bacteria into the cells.

Finally, the inventors tested a possible interaction between EF-Tu and nucleolin at the surface of THP-1 cells, by colocalization experiments (assessed by confocal microscopy) and by a pull-down assay (Fig 4). For confocal experiments, THP-1 cells were incubated with opsonized LVS bacteria. Nucleolin localization (Fig 4A1 and 4B1) was assessed with anti-nucleolin MAb. Localization of bacteria was assessed either with polyclonal anti-LVS (Fig 4A2) or anti-EF-Tu (Fig 4B2) Ab. Merging of nucleolin with LVS (Fig A4 and B4) was observed with both anti-LVS and anti-EF-Tu Abs, demonstrating EF-Tu and nucleolin co-localization. A lower fluorescence intensity of surface nucleolin was observed when anti-EF-Tu Ab was used instead of anti-LVS. This probably results from quenching due to higher concentration of anti-EF-Tu Ab (1/2,000), than that of anti-LVS (1/10,000) Ab (compare A1 to B1).

A pull-down assay was performed by incubating GST or GST-EF-Tu bound on glutathione-sepharose with proteins solubilized from THP-1 cell membranes. Proteins interacting with GST or GST-EF-Tu were submitted to immunoblotting analysis, using anti-nucleolin MAb for detection. As shown in Fig. 4C, a protein doublet at 95 kDa (the expected MW of nucleolin) was detected by anti-nucleolin MAb with GST-EF-Tu, while no detection was observed with GST alone. As control, blotting with polyclonal anti-GST demonstrated that same amounts of GST or GST-EF-Tu were loaded (data not shown). This assay further demonstrated specific interaction between EF-Tu and nucleolin.

### Interaction of nucleolin with F.T. EF-Tu is involved in adhesion and infection of human monocytes

To further evaluate the role of nucleolin - EF-Tu interaction in adhesion and infection of human monocytes by LVS, the inventors tested the effect of HB-19 on binding of LVS to THP-1 cells. HB-19 is a pseudopeptide, which has been previously described to specifically and irreversibly bind to cell-surface nucleolin and to inhibit HIV infection (Said, E. A., 2005). The first measured the number of LVS-GFP bacteria bound to THP-1 cells, using fluorescent microscopy (Fig 5A). Pre-incubation of human monocytes with HB-19 inhibited the binding of LVS-GFP by 70%, in presence of Fetal Calf Serum (FCS). In absence of FCS, HB-19 had no effect on LVS-GFP binding, probably due to serum starvation of cells. Indeed, the active process of HB-19/nucleolin internalization has been shown to be completely blocked in serum-starved cells (Hovanessian, A. G., 2000; Said, E. A., 2005) The inventors also monitored the effect of HB-19 on the number of intracellular bacteria 23 h after LVS infection of THP-1 cells (Fig 5B). Pre-incubation of THP-1 with HB-19 led to an 80% decrease of infection, as compared to THP-1 pre-incubated with anti-GAPDH MAb (used as control of 100% infection). As previously shown (Fig 2), anti-CR3 Mab also decreased the rate of infection by 80%. The inventors therefore examined whether incubation of human cells with both HB-19 and anti-CR3 Mab had an additional or a synergistic effect on the level of infection. Incubation of human monocytes with both HB-19 and anti-CR3 Mab induced a synergistic effect of 95% decrease in the level of infection. Altogether, these results corroborate the notion that interaction between nucleolin and EF-Tu is fundamental for infection of monocytes by *F. tularensis* and that its effect is added to the C3-CR3 receptor interaction.

### The C-terminal Tail of Nucleolin Containing the RGG Repeats Is the Site of Binding to HB-19

In spite of the cationic nature of HB-19, these pseudopeptides do not bind the N-terminal fragment of nucleolin (amino acids 1-308) containing four long stretches of acidic amino acids (A1, A2, A3, A4). On the other hand, HB-19 binds efficiently the C-terminal fragment of nucleolin (309-710).

To illustrate this, truncated constructs of nucleolin corresponding to its N- and C-terminal parts were generated by in vitro transcription/translation in the rabbit reticulocyte lysate system, because the full-length nucleolin and the N-terminal part of nucleolin cannot be expressed in E. coli. [35S]Met/Cys-labeled full-length nucleolin, N-terminal and the C-terminal parts containing amino acids 1-707, 1-308, and 309-707, respectively, produced in the reticulocyte lysate system were incubated with different concentrations of the biotinylated HB-19, and the complex was recovered by avidin-agarose. As expected, the full-length nucleolin was found to bind HB-19. Intriguingly, the N-terminal part of nucleolin containing the acidic stretches did not bind at all, whereas the C-terminal part was bound efficiently to HB-19.

To further characterize the binding domain of HB-19, truncated constructs of the C-terminal part of nucleolin (amino acids 309-710) were generated in E. coli fused with GST as a tag to permit their detection by antibodies against GST. Eight deletion constructs of were generated expressing different RBDs with or without the RGG domain. The HB-19 binding capacity of each construct was then investigated by incubation of crude bacterial extracts with the biotinylated HB-19 (Figure 6). The results demonstrated that the presence of the RGG domain determines the HB-19 binding capacity of a given construct in the C-terminal part of nucleolin. Furthermore, the RGG domain alone is sufficient for binding.

### Specific interaction of F. tularensis EF-Tu with the C-terminal RGG domain of nucleolin.

The pull-down assay showing the binding of nucleolin to GST-EF-Tu bound to glutathione-sepharose suggested a direct interaction mechanism between EF-Tu and nucleolin.

The inventors investigated the capacity of recombinant EF-Tu to bind the p63 peptide, corresponding to the RGG domain, i.e., the last 63 amino acid residues at the C-terminal tail of human nucleolin.

The results shown in Fig. 7A demonstrate that the biotin-labeled p63 peptide binds EF-Tu in a dose dependent manner, with maximum binding occurring at a concentration of 8 µM. The binding of the biotin-labeled p63 peptide was almost completely prevented in the presence of excess unlabeled p63 peptide, thus illustrating that the binding of EF-Tu to the RGG domain of nucleolin is specific (Fig. 7B lanes p63). The specificity of interaction between EF-Tu and the RGG domain was further tested using various peptides: the HB-19 pseudopeptide that binds the RGG domain at the C-terminal tail of nucleolin, the F3 peptide that binds the acidic domains localized at the N-terminal part of nucleolin, and the protease-resistant basic 9Arg peptide that does not interact with nucleolin. Consistent with the binding of EF-Tu to the RGG domain of nucleolin, HB-19 markedly inhibited the binding the biotin-labeled p63 peptide to EF-Tu, whereas the F3 and the 9Arg peptide had no apparent effect (Fig. 7B).

### Activity of nucleolin on the surface of cells treated with different concentrations of HB-19, Nucant 3, Nucant 6, and Nucant 7.

The activity of the cell-surface expressed nucleolin was tested on HeLa P4 cells according to the methods described before (Nisole et al., 2002). Treatment of cells with the different pseudopeptides was at 0.1, 0.2, 0.4 and 0.8 µM concentrations (Figure 9). The Control histogram gives 100 % activity. The results show that the degree of inhibition of nucleolin activity by Nucant 3 is comparable to the effect observed with HB-19. On the other hand, both Nucant 6 and Nucant 7 exert an inhibitory effect that consistently is superior compared to HB-19. Indeed, the IC50 values (Inhibitory Concentration at 50% of the Control) observed for HB-19 and Nucant 3 is 0.1-0.2 µM, whereas that of Nucant 6 and Nucant 7 is less then 0.1 µM. Moreover, Nucant 6 and Nucant 7 at 0.8 µM concentration exert an inhibitory activity that is more than 95%.

### References :

Archambaud, C., Gouin, E., Pizarro-Cerda, J., Cossart, P. & Dussurget, O. (2005) Mol. Microbiol. 56, 383-396.
Balagopal, A., MacFarlane, A. S., Mohapatra, N., Soni, S., Gunn, J. S. & Schlesinger, L. S. (2006) Infect. Immun. 74, 5114-5125.
Balbo, M., Barel, M., Lottin-Divoux, S., Jean, D. & Frade, R. (2005) FEMS Microbiol. Lett. 249, 359-366.
Barbas CF, Bain JD, Hoekstra DM, Lerner RA. (1992), Semisynthetic combinatorial antibody libraries: a chemical solution to the diversity problem. PNAS USA, 89, 4457-4461.
Barel, M., Le Romancer, M. & Frade, R. (2001) J. Immunol. 166, 3167-3173.
Chien CT, Bartel PL, Sternglanz R, Fields S. The two-hybrid system: a method to identify and clone genes for proteins that interact with a protein of interest. Proc Natl Acad Sci U S A. 1991 Nov 1;88(21):9578-82.
Clemens, D. L., Lee, B. Y. & Horwitz, M. A. (2004) Infect. Immun. 72, 3204-3217.
Clemens, D. L., Lee, B. Y. & Horwitz, M. A. (2005) Infect. Immun. 73, 5892-5902.
Dang CV, Barrett J, Villa-Garcia M, Resar LM, Kato GJ, Fearon ER. Intracellular leucine zipper interactions suggest c-Myc hetero-oligomerization. Mol Cell Biol. 1991 Feb;11(2):954-62.
Deng, H., Liu, R., Ellmeier, W., Choe, S., Unutmaz, D., Burkhart, M., Di Marzio, P., Marmon, S., Sutton, R. E., Hill, C. M., Davis, C. B., Peiper, S. C., Schall, T. J., Littman, D. R. & Landau, N. R. (1996) Nature 381, 661-666.
Dennis, D. T., Inglesby, T. V., Henderson, D. A., Bartlett, J. G., Ascher, M. S., Eitzen, E., Fine, A. D., Friedlander, A. M., Hauer, J., Layton, M., Lillibridge, S. R., McDade, J. E., Osterholm, M. T., O'Toole, T., Parker, G., Perl, T. M., Russell, P. K. & Tonat, K. (2001) JAMA 285, 2763-2773.
Edwards PR, Leatherbarrow RJ. Determination of association rate constants by an optical biosensor using initial rate analysis. Anal Biochem. 1997 Mar 1;246(1):1-6.
Fearon ER, Finkel T, Gillison ML, Kennedy SP, Casella JF, Tomaselli GF, Morrow JS, Van Dang C. Karyoplasmic interaction selection strategy: a general strategy to detect protein-protein interactions in mammalian cells. Proc Natl Acad Sci U S A. 1992 Sep 1;89(17):7958-62.
Fields S, Song O. A novel genetic system to detect protein-protein interactions. Nature. 1989 Jul 20;340(6230):245-6.
Fournel, S., Wieckowski, S. Sun, W., Trouche, N., Dumortier, H., Bianco, A., Chaloin, O., Habib, M., Peter, J.C., Schneider, P., Vray, B., Toes, R.E., Offringa, R., Melief, C.J., Hoebeke, J., Guichard, G. C3-symmetric peptide scaffolds are functional mimetics of trimeric CD40L. , Nat Chem Biol. 2005 377-82.
Golovliov, I., Baranov, V., Krocova, Z., Kovarova, H. & Sjostedt, A. (2003) Infect. Immun. 71, 5940-5950.
Harlow E. et al., Antibodies: A Laboratory Manual, Cold Spring Harbor Laboratory, New York, (1988).
Hovanessian, A. G., Puvion-Dutilleul, F., Nisole, S., Svab, J., Perret, E., Deng, J. S. & Krust, B. (2000) Exp. Cell. Res. 261, 312-328.
Kohler and Milstein (1975) Continuous cultures of fused cells secreting antibody of predefined specificity. Nature ;256, 495-7.
Lai, X. H., Golovliov, I. & Sjostedt, A. (2001) Infect. Immun. 69, 4691-4694.
Kurien BT, Scofield RH. Western blotting. Methods. 2006 Apr;38(4):283-93.
Maier, T. M., Havig, A., Casey, M., Nano, F. E., Frank, D. W. & Zahrt, T. C. (2004) Appl. Environ. Microbiol. 70, 7511-7519.
Mauri, D. N., R. Ebner, R. I. Montgomery, K. D. Kochel, T. C. Cheung, G. L.Yu, S. Ruben, 1998.
Morrison SL, Johnson MJ, Herzenberg LA, Oi VT. Chimeric human antibody molecules: mouse antigen-binding domains with human constant region domains. Proc Natl Acad Sci U S A. 1984 Nov;81(21):6851-5.
Murphy, R M. J. Eisenberg, G. H. Cohen, P. G. Spear, C. F. Ware. 1998. LIGHT, a new member of the TNF superfamily, and lymphotoxin are ligands for herpesvirus entry mediator. Immunity 8:21.
Neuberger MS, Williams GT, Fox RO. Recombinant antibodies possessing novel effector functions. Nature. 1984 Dec 13-19;312(5995):604-8.
Nisole, S., Krust, B., Callebaut, C., Guichard, G., Muller, S., Briand, J. P. & Hovanessian, A. G. (1999) J. Biol. Chem. 274, 27875-27884.
Nisole S, Said EA, Mische C, Prevost MC, Krust B, Bouvet P, et al. The anti-HIV pentameric pseudopeptide HB-19 binds the C-terminal end of nucleolin and prevents anchorage of virus particles in the plasma membrane of target cells. J Biol Chem 2002;277:20877-86.
Riechmann L, Clark M, Waldmann H, Winter G Reshaping human antibodies for therapy. Nature. 1988 Mar 24;332(6162):323-7.
Robinson, C. M., Sinclair, J. F., Smith, M. J. & O'Brien, A. D. (2006) Proc. Natl. Acad. Sci. U S A 103, 9667-9672.
Roguska MA, Pedersen JT, Keddy CA, Henry AH, Searle SJ, Lambert JM, Goldmacher VS, Blattler WA, Rees AR, Guild BC. Humanization of murine monoclonal antibodies through variable domain resurfacing. Proc Natl Acad Sci U S A. 1994 Feb 1 ;91 (3):969-73.
Rybicki EP, von Wechmar MB. Enzyme-assisted immune detection of plant virus proteins electroblotted onto nitrocellulose paper. J Virol Methods. 1982 Dec;5(5-6):267-78.
Said AE, Krust B, Nisole S, Briand JP, Hovanessian AG. The anti-HIV cytokine midkine binds the cell-surface-expressed nucleolin as a low affinity receptor. J Biol Chem 2002;277:37492-502.
Said, E. A., Courty, J., Svab, J., Delbe, J., Krust, B. & Hovanessian, A. G. (2005) FEBS J. 272, 4646-4659.
Sanchez-Corral, P., Anton, L. C., Alcolea, J. M., Marques, G., Sanchez, A. & Vivanco, F. (1989) J. Immunol. Methods 122, 105-113.
Schilstra, M. J., Slot, J. W., van der Meide, P. H., Posthuma, G., Cremers, A. F. & Bosch, L. (1984) FEBS Lett. 165, 175-179.
Sinclair, J. F. & O'Brien, A. D. (2002) J. Biol. Chem. 277, 2876-2885.
Sjostedt, A. (2003) Curr. Opin. Microbiol. 6, 66-71.
Shitara K, Nakamura K, Tokutake-Tanaka Y, Fukushima M, Hanai N. A new vector for the high level expression of chimeric antibodies in myeloma cells. J Immunol Methods. 1994 Jan 3;167(1-2):271-8.
Srivastava M., O. W. McBride, P. J. Fleming, H. B. Pollard and A. L. Burns, Genomic organization and chromosomal localization of the human nucleolin gene. J. Biol. Chem. 265 (1990), pp. 14922-14931.
Srivastava M. and H. B. Pollard, Molecular dissection of nucleolin's role in growth and cell proliferation: New insights. FASEB J. 13 (1999), pp. 1911-1922.
Studnicka GM, Soares S, Better M, Williams RE, Nadell R, Horwitz AH. Human-engineered monoclonal antibodies retain full specific binding activity by preserving non-CDR complementarity-modulating residues. Protein Eng. 1994 Jun;7(6):805-14.
Szabo A, Stolz L, Granzow R. Surface plasmon resonance and its use in biomolecular interaction analysis (BIA). Curr Opin Struct Biol. 1995 Oct;5(5):699-705.
Tamada, K., K. Shimozaki, A. I. Chapoval, Y. Zhai, J. Su, S. F. Chen, S. L. Hsieh, S. Nagata, J. Ni, L. Chen. 2000. LIGHT, a TNF-like molecule, costimulates T cell proliferation and is required for dendritic cell-mediated allogeneic T cell response. J. Immunol. 164:4105.
Towbin H, Staehelin T, Gordon J. Electrophoretic transfer of proteins from polyacrylamide gels to nitrocellulose sheets: procedure and some applications. Proc Natl Acad Sci U S A. 1979 Sep;76(9):4350-4.
Vasavada HA, Ganguly S, Germino FJ, Wang ZX, Weissman SM. A contingent replication assay for the detection of protein-protein interactions in animal cells. Proc Natl Acad Sci U S A. 1991 Dec 1;88(23):10686-90.
Waterhouse P, Griffiths AD, Johnson KS, Winter G. (1993) Combinatorial infection and in vivo recombination: a strategy for making large phage antibody repertoires. Nucleic Acids Research, 21, 2265-2266.
Yu, K. Y., B. Kwon, J. Ni, Y. Zhai, R. Ebner, B. S. Kwon. 1999. A newly identified member of tumor necrosis factor receptor superfamily (TR6) suppresses LIGHT-mediated apoptosis. J. Biol. Chem. 274:13733.

## Claims

1. Use of an inhibitor of the interaction between the bacterial elongation factor Tu (EF-Tu) of *Francisella tularensis* and nucleolin for the manufacture of a medicament for preventing or treating tularemia.

2. The use according to claim 1 wherein said inhibitor is a ligand of nucleolin

3. The use according to claim 2, wherein the ligand of nucleolin binds to the RGG domain of nucleolin.

4. The use according to claim 2 or 3, wherein said ligand of nucleolin is a multivalent synthetic compound which comprises or is constituted of a template on which at least 3 pseudopeptides are grafted, said pseudopeptide having the formula 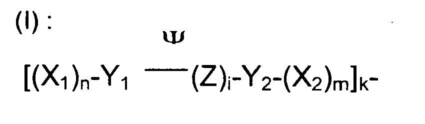 wherein
X₁ and X₂ independently represents any amino acid;
Y₁ and Y₂ independently are amino acids having a basic lateral chain;
Z is selected from the group consisting of :
- proline, optionnally substituted in γ, β or δ by hydroxyl, amino, C₁-C₁₀ alkyl, C₁-C₁₀ alkenyl, C₁-C₁₀ alkynyl, C₅-C₁₂ aryl, C₅-C₁₄ aralkyl, C₅-C₁₂ heteroaryl group, such group being optionnally substituted by 1 to 6 substituents selected from the group consisting of halogen, NO₂, OH, C₁-C₄ alkyl, NH₂, CN, trihalomethyl, C₁-C₄ acyloxy, C₁-C₄dialkylamino, guanidino, thiol;
- (natural or not natural) N-alkylated amino acid;
- dialkylated amino acid;
- dialkylated cyclic amino acid; or
- pipecolic acid or one derivative thereof;
n and i independently are 0 or 1 ;
m is an integer between 0 and 3 ;
k is an integer superior or equal to 3 ; and
Ψ represents a modified peptide bond, significantly more resistant to at least one protease than a normal peptide bond.

5. The use according to claim 4, wherein the template is selected from the group consisting of a linear peptide, a cyclic peptide, and a peptoide that is linear or cyclic.

6. The use of claim 5, wherein the template is a linear peptide having a sequence selected from the group consisting of KKKGPKEKGC (SEQ ID NO :3), KKKKGC (SEQ ID NO :4), KKKKGPKKKKGA (SEQ ID NO :5), KKKGPKEKAhxCONH₂ (SEQ ID NO :6, wherein Ahx represents hexanoïc amino acid and CONH₂ represents an amide function instead of the acid function, and a sequence which is constituted of 2 to 4 motifs KAKPG (SEQ ID NO :7).

7. The use of any of claims 4 to 6, wherein Ψ is selected from the group consisting of a reduced bond that is (-CH₂NH-) or (-CH₂N-) when the bond involves a secondary amino group, retro inverso (-NHCO-), methylene oxy (-CH₂-O-), thiomethylene (-CH₂-S-), carba (-CH₂CH₂-), cetomethylene (-CO-CH₂-), hydroxyethylene (-CHOH-CH₂), (-N-N-), E-alcene and (-CH=CH-) bond.

8. The use of any of claims 4 to 7, wherein both n and m are 0.

9. The use of any of claims 4 to 8, wherein k is an integer between 3 and 8.

10. The use of any of claims 4 to 9, wherein I=1 and Z is proline.

11. The use of any of claims 4 to 10, wherein Y₁ is lysine (K) and Y₂ is arginine (R).

12. The use according to claim 3, wherein the ligand of nucleolin is selected from the group consisting of: 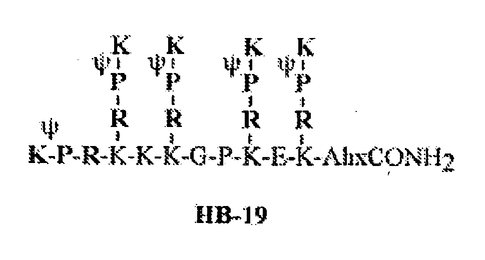 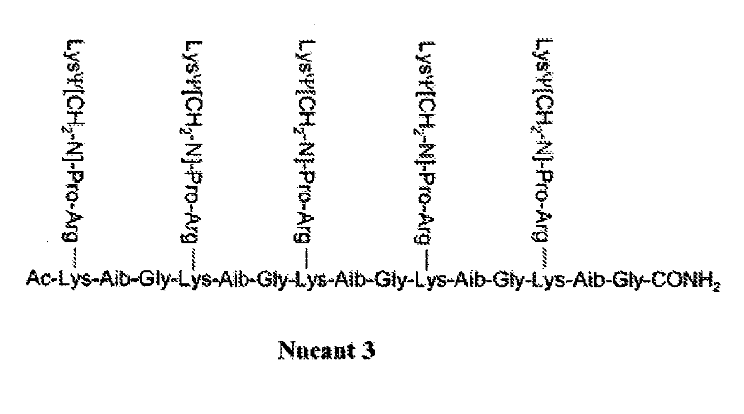 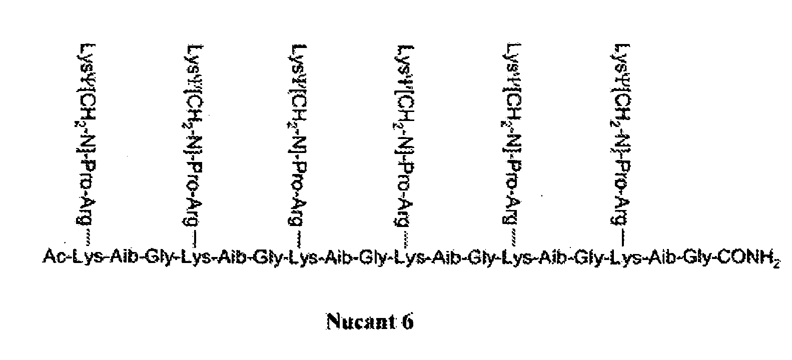 and 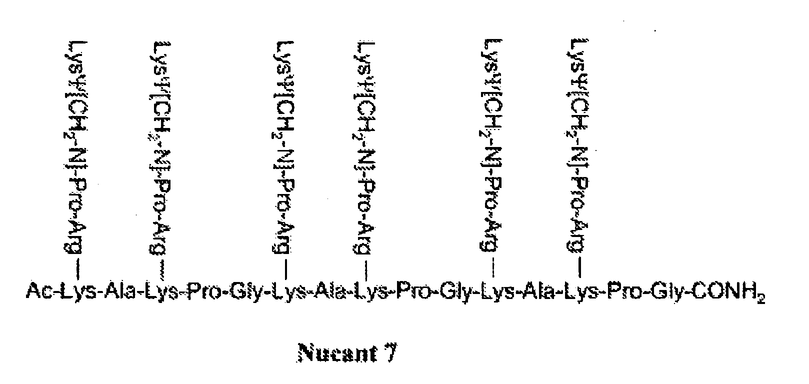 wherein Ψ(CH₂-N) stands for a reduced peptide bond and AhxCONH₂ represents aminohexanamide, and Aib represents 2-amino-isobutyrique acid.

13. The use according to claim 1, wherein said inhibitor is an antibody against the RGG domain of nucleolin.

14. The use according to claim 1 wherein said inhibitor is a ligand of the bacterial elongation factor Tu (EF-Tu) of *Fransiciella tularensis.*

15. The use according to claim 14, wherein said ligand of EF-Tu is an antibody directed against EF-Tu.

16. Use of bacterial elongation factor Tu (EF-Tu) of F. tularensis for the manufacture of an immunogenic composition for preventing or treating tularemia.

17. An immunogenic composition comprising bacterial elongation factor Tu (EF-Tu) of F. tularensis.

18. A pharmaceutical composition comprising an antibody against bacterial
elongation factor Tu (EF-Tu) of F. tularensis.

19. An *in vitro* method for screening compounds useful for preventing or treating Tularemia, comprising contacting a compound with bacterial elongation factor Tu (EF-Tu) of F. tularensis and nucleolin or the RGG domain of nucleolin, and determining the ability of the compound to inhibit the interaction between EF-Tu and nucleolin or the RGG domain of nucleolin, wherein inhibition of the interaction is indicative of a compound useful for preventing or treating tularemia.

## Patentansprüche

1. Verwendung eines Inhibitors der Wechselwirkung zwischen dem bakteriellen Elongationsfaktor Tu (EF-Tu) von *Francisella tularensis* und Nucleolin zur Herstellung eines Arzneimittels zur Prävention oder zur Behandlung von Tularämie.

2. Verwendung nach Anspruch 1, wobei der Inhibitor ein Ligand von Nucleolin ist.

3. Verwendung nach Anspruch 2, wobei der Ligand von Nucleolin an die RGG-Domäne von Nucleolin bindet.

4. Verwendung nach Anspruch 2 oder 3, wobei der Ligand von Nucleolin eine multivalente synthetische Verbindung ist, welche eine Matrize enthält oder aus einer Matrize zusammengesetzt ist, auf der wenigstens drei Pseudopeptide gepfropft sind, wobei das Pseudopeptid die Formel (I) aufweist: 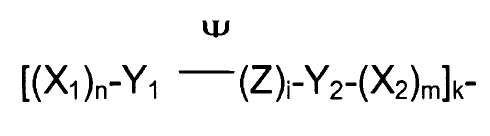 worin
X₁ und X₂, unabhängig voneinander, eine Aminosäure bedeuten,
Y₁ und Y₂, unabhängig voneinander, Aminosäuren mit einer basischen Seitenkette sind,
Z aus der Gruppe ausgewählt wird, welche besteht aus:
- Prolin, optional substituiert in γ, β oder δ durch Hydroxyl-, Amino-, C₁-C₁₀-Alkyl-, C₁-C₁₀-Alkenyl-, C₁-C₁₀-Alkynyl-, C₅-C₁₂-Aryl-, C₅-C₁₄-Aralkyl-, C₅-C₁₂-Heteroarylgruppe, wobei eine solche Gruppe optional substituiert ist mit 1 bis 6 Substituenten ausgewählt aus der Gruppe bestehend aus Halogen, NO₂, OH, C₁-C₄-Alkyl, NH₂, CN, Trihalomethyl, C₁-C₄-Acyloxy, C₁-C₄-Dialkylamino, Guanidino, Thiol,
- (natürlicher oder nicht natürlicher) N-alkylierter Aminosäure,
- dialkylierter Aminosäure,
- dialkylierter zyklischer Aminosäure oder
- Pipecolinsäure oder einem Derivat hiervon,
n und i, unabhängig voneinander, 0 oder 1 sind,
m eine ganze Zahl zwischen 0 und 3 ist,
k eine ganze Zahl von größer als 3 ist und
Ψ eine modifizierte Peptidbindung bedeutet, welche gegenüber wenigstens einer Protease beträchtlich resistenter ist als eine normale Peptidbindung.

5. Verwendung nach Anspruch 4, wobei die Matrize aus der Gruppe ausgewählt wird, welche aus einem linearen Peptid, einem zyklischen Peptid und einem Peptoid, welches linear oder zyklisch ist, besteht.

6. Verwendung nach Anspruch 5, wobei die Matrize ein lineares Peptid mit einer Sequenz ist, welche aus der Gruppe ausgewählt wird, welche aus KKKGPKEKGC (SEQ ID NO :3), KKKKGC (SEQ ID NO :4), KKKKGPKKKKGA (SEQ ID NO :5), KKKGPKEKAhxCONH₂ (SEQ ID NO :6), wobei Ahx Hexansäure bezeichnet und CONH₂ eine Amidfunktion anstelle der Säurefunktion bezeichnet, und einer Sequenz, welche aus 2 bis 4 Motiven KAKPG (SEQ ID NO :7) zusammengesetzt ist, besteht.

7. Verwendung nach einem der Ansprüche 4 bis 6, wobei Ψ aus der Gruppe ausgewählt wird, welche aus einer reduzierten Bindung besteht, welche eine (-CH₂NH-) oder (-CH₂N-), wenn die Bindung eine sekundäre Aminogruppe umfasst, retro inverso (-NHCO-), Methylenoxy (-CH₂-O-), Thiomethylen (-CH₂-S-), Carba (-CH₂CH₂-), Cetomethylen (-CO-CH₂-), Hydroxyethylen (-CHOH-CH₂-), (-N-N-), E-Alken oder (-CH=CH-) Bindung ist.

8. Verwendung nach einem der Ansprüche 4 bis 7, wobei sowohl n als auch m 0 sind.

9. Verwendung nach einem der Ansprüche 4 bis 8, wobei k eine ganze Zahl zwischen 3 und 8 ist.

10. Verwendung nach einem der Ansprüche 4 bis 9, wobei 1=1 ist und Z Prolin ist.

11. Verwendung nach einem der Ansprüche 4 bis 10, wobei Y₁ Lysin (K) ist und Y₂ Arginin (R) ist.

12. Verwendung nach Anspruch 3, wobei der Ligand von Nucleolin aus der Gruppe ausgewählt wird, welche besteht aus: 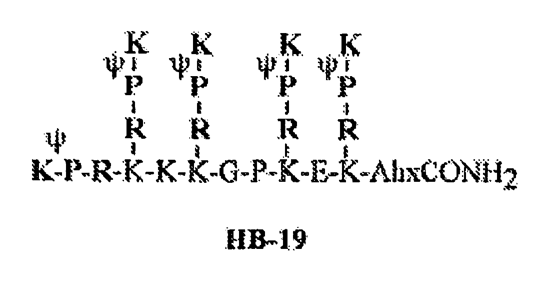 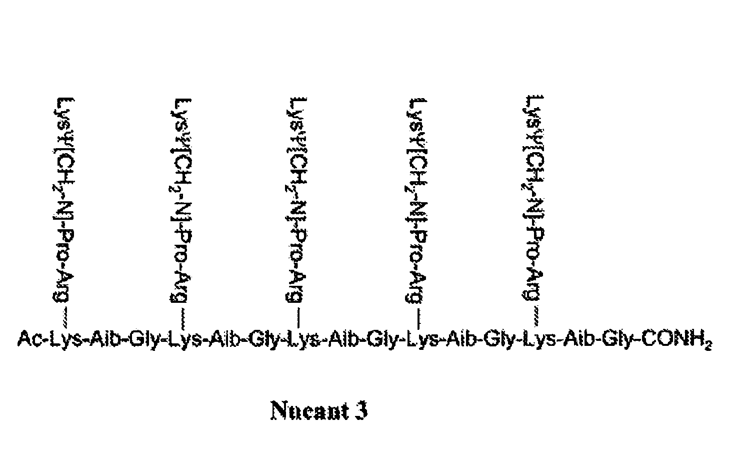 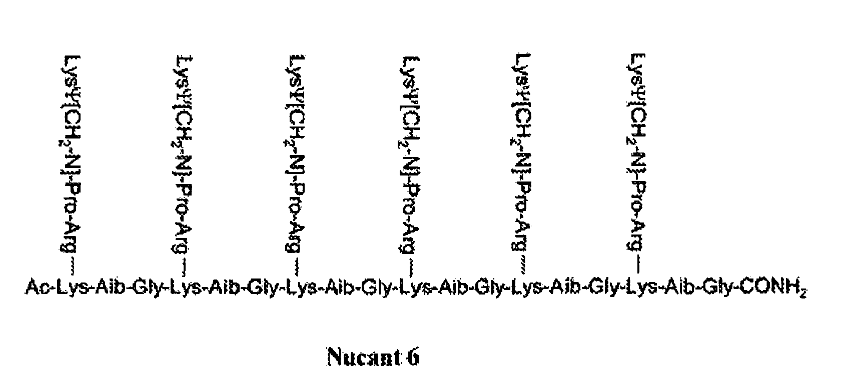 und 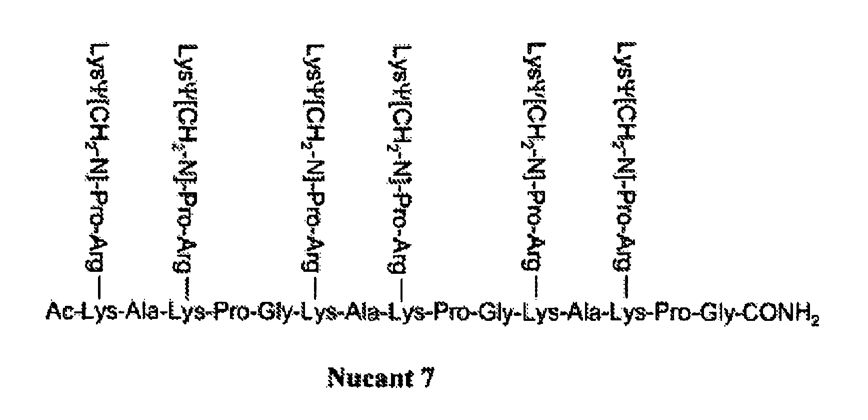 worin Ψ(CH₂-N) für eine reduzierte Peptidbindung steht und AhxCONH₂ Aminohexanamid bedeutet und Aib 2-Amino-isobuttersäure bedeutet.

13. Verwendung nach Anspruch 1, wobei der Inhibitor ein Antikörper gegen die RGG-Domäne von Nucleolin ist.

14. Verwendung nach Anspruch 1, wobei der Inhibitor ein Ligand des bakteriellen Elongationsfaktors Tu (EF-Tu) von *Fransiciella tularensis* ist.

15. Verwendung nach Anspruch 14, wobei der Ligand EF-Tu ein Antikörper ist, welcher gegen EF-Tu gerichtet ist.

16. Verwendung des bakteriellen Elongationsfaktors Tu (EF-Tu) von *F. tularensis* zur Herstellung eines Arzneimittels einer immunogenen Zusammensetzung zur Prävention oder Behandlung von Tularämie.

17. Eine immunogene Zusammensetzung, welche den bakteriellen Elongationsfaktor Tu (EF-Tu) von *F. tularensis* enthält.

18. Pharmazeutische Zusammensetzung, welche einen Antikörper gegen den bakteriellen Elongationsfaktor Tu (EF-Tu) von *F. tularensis* enthält.

19. Ein *in vitro* Verfahren zum Screenen von Verbindungen, welche zur Prävention oder Behandlung von Tularämie geeignet sind, umfassend das Kontaktieren einer Verbindung mit einem bakteriellen Elongationsfaktor Tu (EF-Tu) von *F. tularensis* und Nucleolin oder der RGG-Domäne von Nucleolin, sowie Bestimmen der Fähigkeit der Verbindung, die Wechselwirkungen zwischen EF-Tu und Nucleolin oder der RGG-Domäne von Nucleolin zu inhibieren, wobei die Inhibition der Wechselwirkung für eine Verbindung indikativ ist, welche zur Prävention oder zur Behandlung von Tularämie geeignet ist.

## Revendications

1. Utilisation d'un inhibiteur de l'interaction entre le facteur d'allongement bactérien Tu (EF-Tu) de *Francisiella tularensis* et la nucléoline pour la production d'un médicament destiné à prévenir ou à traiter la tularémie.

2. Utilisation selon la revendication 1, dans laquelle ledit inhibiteur est un ligand de la nucléoline.

3. Utilisation selon la revendication 2, dans laquelle le ligand de la nucléoline se lie au domaine RGG de la nucléoline.

4. Utilisation selon la revendication 2 ou 3, dans laquelle ledit ligand de la nucléoline est un composé synthétique multivalent qui comprend ou est constitué d'une matrice sur laquelle au moins 3 pseudopeptides sont greffés, ledit pseudopeptide ayant la formule 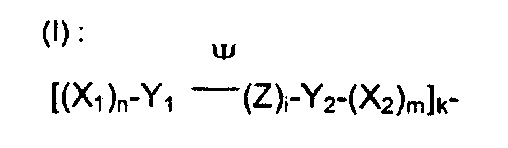 dans laquelle X₁ et X₂ représentent indépendamment un acide aminé ;
Y₁ et Y₂ sont indépendamment des acides aminés ayant une chaîne latérale basique ;
Z est choisi dans le groupe comprenant :
- la proline éventuellement substituée en γ, β ou δ par un groupe hydroxyle, amino, alkyle en C₁ à C₁₀, alcényle en C₁ à C₁₀, alcynyle en C₁ à C₁₀, aryle en C₅ à C₁₂, aralkyle en C₅ à C₁₄, hétéroaryle en C₅ à C₁₂, ce groupe étant éventuellement substitué par 1 à 6 substituants choisis dans le groupe comprenant un atome d'halogène, NO₂, OH, un groupe alkyle en C₁ à C₄, NH₂, CN, trihalogénométhyle, acyloxy en C₁ à C₄, dialkylamino en C₁ à C₄, guanidino, thiol ;
- un acide aminé N-alkylé (naturel ou non naturel) ;
- un acide aminé dialkylé ;
- un acide aminé cyclique dialkylé ; ou
- un acide pipécolique ou l'un de ses dérivés ;
n et i sont indépendamment 0 ou 1 ;
m est un nombre entier entre 0 et 3 ;
k est un nombre entier supérieur ou égal à 3 ; et
Ψ représente une liaison peptide modifiée, significativement plus résistante à au moins une protéase qu'une liaison peptide normale.

5. Utilisation selon la revendication 4, dans laquelle la matrice est choisie dans le groupe comprenant un peptide linéaire, un peptide cyclique et un peptoïde qui est linéaire ou cyclique.

6. Utilisation selon la revendication 5, dans laquelle la matrice est un peptide linéaire ayant une séquence choisie dans le groupe comprenant KKKGPKEKGC (SEQ ID N° : 3), KKKKGC (SEQ ID N° : 4), KKKKGPKKKKGA (SEQ ID N° : 5), KKKGPKEKAhxCONH₂ (SEQ ID N° : 6), dans laquelle Ahx représente l'acide aminé hexanoïque et CONH₂ représente une fonction amide à la place de la fonction acide et une séquence qui est constituée de 2 à 4 motifs KAKPG (SEQ ID n° : 7).

7. Utilisation selon l'une quelconque des revendications 4 à 6, dans laquelle Ψ est choisi dans le groupe comprenant une liaison réduite qui est (-CH₂NH-), ou (-CH₂N-) lorsque la liaison comprend un groupe amino secondaire, rétro-inversé (-NHCO-), méthylène-oxy (-CH₂-O-), thiométhylène (-CH₂-S-), carba (-CH₂CH₂-), cétométhylène (-CO-CH₂-), hydroxyéthylène (-CHOH-CH₂-), (-N-N-), E-alcène et (-CH=CH-).

8. Utilisation selon l'une quelconque des revendications 4 à 7, dans laquelle les deux n et m valent 0.

9. Utilisation selon l'une quelconque des revendications 4 à 8, dans laquelle k est un nombre entier entre 3 et 8.

10. Utilisation selon l'une quelconque des revendications 4 à 9, dans laquelle I = 1 et Z est la proline.

11. Utilisation selon l'une quelconque des revendications 4 à 10, dans laquelle Y₁ est la lysine (K) et Y₂ est l'arginine (R).

12. Utilisation selon la revendication 3, dans laquelle le ligand de la nucléoline est choisi dans le groupe comprenant : 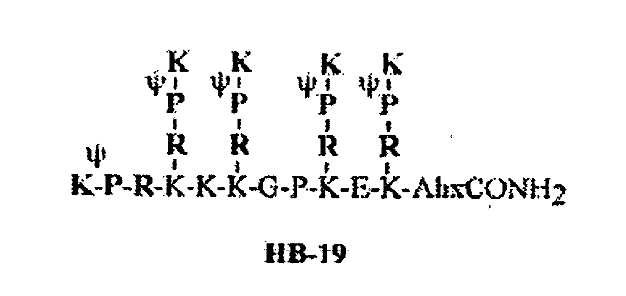 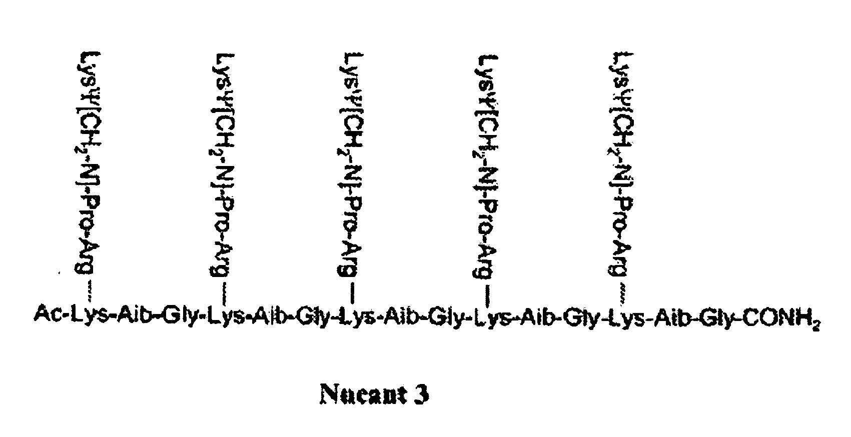 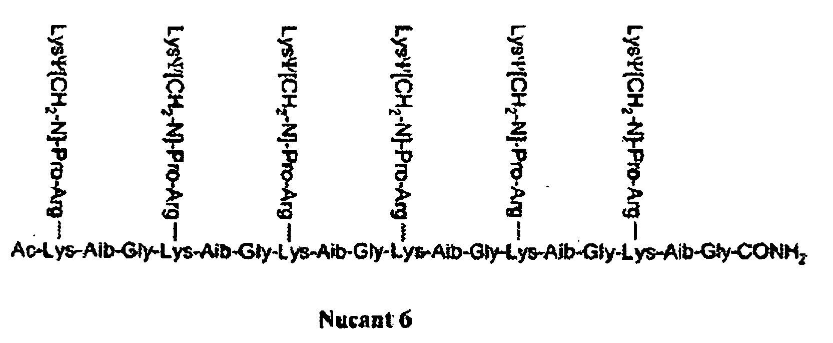 et 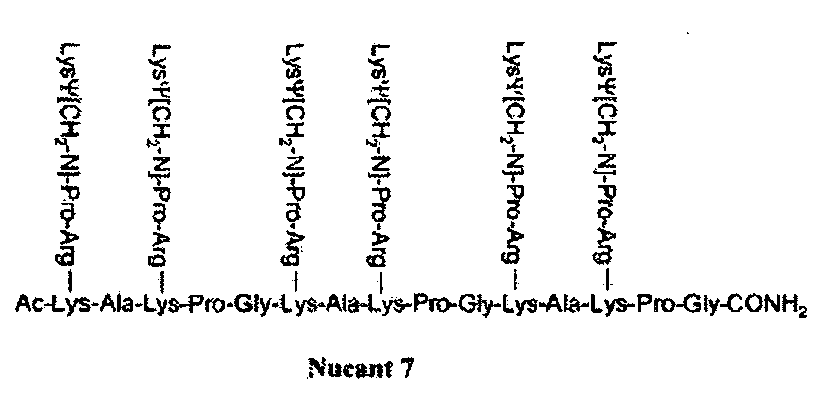 où Ψ(CH₂-N) désigne une liaison peptide réduite et AhxCONH₂ représente l'aminohexanamide et Aib représente l'acide 2-amino-isobutyrique.

13. Utilisation selon la revendication 1, dans laquelle ledit inhibiteur est un anticorps contre le domaine RGG de la nucléoline.

14. Utilisation selon la revendication 1, dans laquelle ledit inhibiteur est un ligand du facteur d'allongement bactérien Tu (EF-Tu) de *Fransiciella tularensis*.

15. Utilisation selon la revendication 14, dans laquelle ledit ligand de EF-Tu est un anticorps dirigé contre EF-Tu.

16. Utilisation du facteur d'allongement bactérien Tu (EF-Tu) de *F. tularensis* pour la production d'une composition immunogène pour prévenir ou traiter la tularémie.

17. Composition immunogène comprenant le facteur d'allongement bactérien Tu (EF-Tu) de *F. tularensis*.

18. Composition pharmaceutique comprenant un anticorps contre le facteur d'allongement bactérien Tu (EF-Tu) de *F. tularensis*.

19. Procédé *in vitro* de détection de composés utiles pour prévenir ou traiter la tularémie, comprenant la mise en contact d'un composé avec le facteur d'allongement bactérien Tu (EF-Tu) de *F. tularensis* et la nucléoline ou le domaine RGG de la nucléoline, et la détermination de la capacité du composé à inhiber l'interaction entre EF-Tu et la nucléoline ou le domaine RGG de la nucléoline, l'inhibition de l'interaction étant indicative d'un composé utile pour prévenir ou traiter la tularémie.


### SEQUENCE LISTING

<110> INSERM
<120> Methods for preventing or treating tularemia
<130> B0590/EP
<160> 20
<170> PatentIn version 3.3
<210> 1
   <211> 394
   <212> PRT
   <213> Francisella tularensis
<400> 1
   Met Ala Lys Glu Lys Phe Glu Arg Ser Lys Pro His Val Asn Val Gly
   1               5                   10                  15
   Thr Ile Gly His Val Asp His Gly Lys Thr Thr Leu Thr Ala Ala Ile
               20                  25                  30
   Thr Lys Val Met Ala Glu Lys Asn Gly Gly Met Ala Arg Lys Phe Asp
           35                  40                  45
   Glu Ile Asp Ser Ala Pro Glu Glu Lys Ala Arg Gly Ile Thr Ile Asn
       50                  55                  60
   Thr Ser His Val Glu Tyr Glu Ser Pro Asn Arg His Tyr Ala His Val
   65                  70                  75                  80
   Asp Cys Pro Gly His Ala Asp Tyr Val Lys Asn Met Ile Thr Gly Ala
                   85                  90                  95
   Ala Gln Met Asp Gly Ala Ile Leu Val Cys Ser Ala Ala Asp Gly Pro
               100                 105                 110
   Met Pro Gln Thr Arg Glu His Ile Leu Leu Ser Arg Gln Val Gly Val
           115                 120                 125
   Pro Lys Ile Val Val Phe Leu Asn Lys Cys Asp Met Val Asp Asp Glu
       130                 135                 140
   Glu Leu Leu Glu Leu Val Glu Met Glu Val Arg Glu Leu Leu Asp Gln
   145                 150                 155                 160
   Tyr Glu Phe Pro Gly Asp Asp Thr Pro Val Ile Met Gly Ser Ala Leu
                   165                 170                 175
   Arg Ala Ile Glu Gly Asp Glu Ala Tyr Val Glu Lys Ile Val Glu Leu
               180                 185                 190
   Val Gln Ala Met Asp Asp Tyr Ile Pro Ala Pro Glu Arg Asp Thr Glu
           195                 200                 205
   Lys Pro Phe Ile Leu Pro Ile Glu Asp Val Phe Ser Ile Ser Gly Arg
       210                 215                 220
   Gly Thr Val Val Thr Gly Arg Ile Glu Arg Gly Val Val Asn Val Gly
   225                 230                 235                 240
   Asp Glu Val Glu Val Val Gly Ile Arg Pro Thr Gln Lys Thr Thr Val
                   245                 250                 255
   Thr Gly Val Glu Met Phe Arg Lys Leu Leu Asp Arg Gly Glu Ala Gly
               260                 265                 270
   Asp Asn Val Gly Ile Leu Val Arg Gly Leu Lys Arg Asp Asp Val Glu
           275                 280                 285
   Arg Gly Gln Val Leu Cys Lys Pro Gly Ser Ile Lys Pro His Thr Lys
       290                 295                 300
   Phe Glu Ala Glu Val Tyr Val Leu Ser Lys Glu Glu Gly Gly Arg His
   305                 310                 315                 320
   Thr Pro Phe Phe Lys Gly Tyr Arg Pro Gln Phe Tyr Phe Arg Thr Thr
                   325                 330                 335
   Asp Ile Thr Gly Ala Val Glu Leu Pro Glu Gly Val Glu Met Val Met
               340                 345                 350
   Pro Gly Asp Asn Val Lys Met Thr Ile Thr Leu Ile Asn Pro Ile Ala
           355                 360                 365
   Met Asp Glu Gly Leu Arg Phe Ala Ile Arg Glu Gly Gly Arg Thr Val
       370                 375                 380
   Gly Ala Gly Val Val Ala Lys Ile Ile Glu
   385                 390
<210> 2
   <211> 710
   <212> PRT
   <213> homo sapiens
<400> 2
   Met Val Lys Leu Ala Lys Ala Gly Lys Asn Gln Gly Asp Pro Lys Lys
   1               5                   10                  15
   Met Ala Pro Pro Pro Lys Glu Val Glu Glu Asp Ser Glu Asp Glu Glu
               20                  25                  30
   Met Ser Glu Asp Glu Glu Asp Asp Ser Ser Gly Glu Glu Val Val Ile
           35                  40                  45
   Pro Gln Lys Lys Gly Lys Lys Ala Ala Ala Thr Ser Ala Lys Lys Val
       50                  55                  60
   Val Val Ser Pro Thr Lys Lys Val Ala Val Ala Thr Pro Ala Lys Lys
   65                  70                  75                  80
   Ala Ala Val Thr Pro Gly Lys Lys Ala Ala Ala Thr Pro Ala Lys Lys
                   85                  90                  95
   Thr Val Thr Pro Ala Lys Ala Val Thr Thr Pro Gly Lys Lys Gly Ala
               100                 105                 110
   Thr Pro Gly Lys Ala Leu Val Ala Thr Pro Gly Lys Lys Gly Ala Ala
           115                 120                 125
   Ile Pro Ala Lys Gly Ala Lys Asn Gly Lys Asn Ala Lys Lys Glu Asp
       130                 135                 140
   Ser Asp Glu Glu Glu Asp Asp Asp Ser Glu Glu Asp Glu Glu Asp Asp
   145                 150                 155                 160
   Glu Asp Glu Asp Glu Asp Glu Asp Glu Ile Glu Pro Ala Ala Met Lys
                   165                 170                 175
   Ala Ala Ala Ala Ala Pro Ala Ser Glu Asp Glu Asp Asp Glu Asp Asp
               180                 185                 190
   Glu Asp Asp Glu Asp Asp Asp Asp Asp Glu Glu Asp Asp Ser Glu Glu
           195                 200                 205
   Glu Ala Met Glu Thr Thr Pro Ala Lys Gly Lys Lys Ala Ala Lys Val
       210                 215                 220
   Val Pro Val Lys Ala Lys Asn Val Ala Glu Asp Glu Asp Glu Glu Glu
   225                 230                 235                 240
   Asp Asp Glu Asp Glu Asp Asp Asp Asp Asp Glu Asp Asp Glu Asp Asp
                   245                 250                 255
   Asp Asp Glu Asp Asp Glu Glu Glu Glu Glu Glu Glu Glu Glu Glu Pro
               260                 265                 270
   Val Lys Glu Ala Pro Gly Lys Arg Lys Lys Glu Met Ala Lys Gln Lys
           275                 280                 285
   Ala Ala Pro Glu Ala Lys Lys Gln Lys Val Glu Gly Thr Glu Pro Thr
       290                 295                 300
   Thr Ala Phe Asn Leu Phe Val Gly Asn Leu Asn Phe Asn Lys Ser Ala
   305                 310                 315                 320
   Pro Glu Leu Lys Thr Gly Ile Ser Asp Val Phe Ala Lys Asn Asp Leu
                   325                 330                 335
   Ala Val Val Asp Val Arg Ile Gly Met Thr Arg Lys Phe Gly Tyr Val
               340                 345                 350
   Asp Phe Glu Ser Ala Glu Asp Leu Glu Lys Ala Leu Glu Leu Thr Gly
           355                 360                 365
   Leu Lys Val Phe Gly Asn Glu Ile Lys Leu Glu Lys Pro Lys Gly Lys
       370                 375                 380
   Asp Ser Lys Lys Glu Arg Asp Ala Arg Thr Leu Leu Ala Lys Asn Leu
   385                 390                 395                 400
   Pro Tyr Lys Val Thr Gln Asp Glu Leu Lys Glu Val Phe Glu Asp Ala
                   405                 410                 415
   Ala Glu Ile Arg Leu Val Ser Lys Asp Gly Lys Ser Lys Gly Ile Ala
               420                 425                 430
   Tyr Ile Glu Phe Lys Thr Glu Ala Asp Ala Glu Lys Thr Phe Glu Glu
           435                 440                 445
   Lys Gln Gly Thr Glu Ile Asp Gly Arg Ser Ile Ser Leu Tyr Tyr Thr
       450                 455                 460
   Gly Glu Lys Gly Gln Asn Gln Asp Tyr Arg Gly Gly Lys Asn Ser Thr
   465                 470                 475                 480
   Trp Ser Gly Glu Ser Lys Thr Leu Val Leu Ser Asn Leu Ser Tyr Ser
                   485                 490                 495
   Ala Thr Glu Glu Thr Leu Gln Glu Val Phe Glu Lys Ala Thr Phe Ile
               500                 505                 510
   Lys Val Pro Gln Asn Gln Asn Gly Lys Ser Lys Gly Tyr Ala Phe Ile
           515                 520                 525
   Glu Phe Ala Ser Phe Glu Asp Ala Lys Glu Ala Leu Asn Ser Cys Asn
       530                 535                 540
   Lys Arg Glu Ile Glu Gly Arg Ala Ile Arg Leu Glu Leu Gln Gly Pro
   545                 550                 555                 560
   Arg Gly Ser Pro Asn Ala Arg Ser Gln Pro Ser Lys Thr Leu Phe Val
                   565                 570                 575
   Lys Gly Leu Ser Glu Asp Thr Thr Glu Glu Thr Leu Lys Glu Ser Phe
               580                 585                 590
   Asp Gly Ser Val Arg Ala Arg Ile Val Thr Asp Arg Glu Thr Gly Ser
           595                 600                 605
   Ser Lys Gly Phe Gly Phe Val Asp Phe Asn Ser Glu Glu Asp Ala Lys
       610                 615                 620
   Ala Ala Lys Glu Ala Met Glu Asp Gly Glu Ile Asp Gly Asn Lys Val
   625                 630                 635                 640
   Thr Leu Asp Trp Ala Lys Pro Lys Gly Glu Gly Gly Phe Gly Gly Arg
                   645                 650                 655
   Gly Gly Gly Arg Gly Gly Phe Gly Gly Arg Gly Gly Gly Arg Gly Gly
               660                 665                 670
   Arg Gly Gly Phe Gly Gly Arg Gly Arg Gly Gly Phe Gly Gly Arg Gly
           675                 680                 685
   Gly Phe Arg Gly Gly Arg Gly Gly Gly Gly Asp His Lys Pro Gln Gly
       690                 695                 700
   Lys Lys Thr Lys Phe Glu
   705                 710
<210> 3
   <211> 10
   <212> PRT
   <213> Artificial sequence
<220>
   <223> artificial
<400> 3
   Lys Lys Lys Gly Pro Lys Glu Lys Gly Cys
   1               5                   10
<210> 4
   <211> 6
   <212> PRT
   <213> artificial sequence
<220>
   <223> artificial
<400> 4
   Lys Lys Lys Lys Gly Cys
   1               5
<210> 5
   <211> 12
   <212> PRT
   <213> artificial sequence
<220>
   <223> artificial
<400> 5
   Lys Lys Lys Lys Gly Pro Lys Lys Lys Lys Gly Ala
   1               5                   10
<210> 6
   <211> 8
   <212> PRT
   <213> artificial sequence
<220>
   <223> artificial
<220>
   <221> BINDING
   <222> (8)..(8)
   <223> "AhxCONH2= (2S)-2-aminohexanamide
<400> 6
   Lys Lys Lys Gly Pro Lys Glu Lys
   1               5
<210> 7
   <211> 5
   <212> PRT
   <213> artificial sequence
<220>
   <223> artificial
<400> 7
   Lys Ala Lys Pro Gly
   1               5
<210> 8
   <211> 15
   <212> PRT
   <213> artificial sequence
<220>
   <223> artificial
<220>
   <221> MOD RES
   <222> (1)..(1)
   <223> ACETYLATION
<220>
   <221> MOD_RES
   <222> (15)..(15)
   <223> AMIDATION
<400> 8
   Lys Ala Lys Pro Gly Lys Ala Lys Pro Gly Lys Ala Lys Pro Gly
   1               5                   10                  15
<210> 9
   <211> 8
   <212> PRT
   <213> artificial sequence
<220>
   <223> artificial
<220>
   <221> BINDING
   <222> (8)..(8)
   <223> "AhxCONH2=(2S)-2-aminohexanamide"
<400> 9
   Lys Lys Lys Gly Pro Lys Glu Lys
   1               5
<210> 10
   <211> 15
   <212> PRT
   <213> artificial sequence
<220>
   <223> artificial
<220>
   <221> MOD RES
   <222> (1)..(1)
   <223> ACETYLATION
<220>
   <221> MOD_RES
   <222> (15)..(15)
   <223> AMIDATION
<400> 10
   Lys Ala Lys Pro Gly Lys Ala Lys Pro Gly Lys Ala Lys Pro Gly
   1               5                   10                  15
<210> 11
   <211> 10
   <212> PRT
   <213> artificial sequence
<220>
   <223> artificial
<220>
   <221> BINDING
   <222> (1)..(1)
   <223> "Aib=2-amino-isobutyric acid"
<220>
   <221> BINDING
   <222> (1)..(2)
   <223> "Aib=2-amino-isobutyric acid"
<220>
   <221> BINDING
   <222> (2)..(3)
   <223> "Aib=2-amino-isobutyric acid"
<220>
   <221> BINDING
   <222> (3)..(4)
   <223> "Aib=2-amino-isobutyric acid"
<220>
   <221> BINDING
   <222> (4)..(5)
   <223> "Aib=2-amino-isobutyric acid"
<220>
   <221> BINDING
   <222> (5)..(6)
   <223> "Aib=2-amino-isobutyric acid"
<220>
   <221> BINDING
   <222> (6)..(7)
   <223> "Aib=2-amino-isobutyric acid"
<220>
   <221> BINDING
   <222> (7)..(8)
   <223> "Aib=2-amino-isobutyric acid"
<220>
   <221> BINDING
   <222> (8)..(9)
   <223> "Aib=2-amino-isobutyric acid"
<220>
   <221> BINDING
   <222> (9)..(10)
   <223> "Aib=2-amino-isobutyric acid"
<400> 11
   Lys Gly Lys Gly Lys Gly Lys Gly Lys Gly
   1               5                   10
<210> 12
   <211> 10
   <212> PRT
   <213> artificial sequence
<220>
   <223> artificial
<220>
   <221> BINDING
   <222> (1)..(2)
   <223> "Aib=2-amino-isobutyric acid"
<220>
   <221> BINDING
   <222> (3)..(4)
   <223> "Aib=2-amino-isobutyric acid"
<220>
   <221> BINDING
   <222> (5)..(6)
   <223> "Aib=2-amino-isobutyric acid"
<220>
   <221> BINDING
   <222> (7)..(8)
   <223> "Aib=2-amino-isobutyric acid"
<220>
   <221> BINDING
   <222> (9)..(10)
   <223> "Aib=2-amino-isobutyric acid"
<400> 12
   Lys Gly Lys Gly Lys Gly Lys Gly Lys Gly
   1               5                   10
<210> 13
   <211> 10
   <212> PRT
   <213> artificial sequence
<220>
   <223> artificial
<220>
   <221> BINDING
   <222> (1)..(1)
   <223> "Ac-Aib =acetyl-2-amino-isobutyric acid""
<220>
   <221> BINDING
   <222> (1)..(2)
   <223> "Aib=2-amino-isobutyric acid"
<220>
   <221> BINDING
   <222> (2)..(3)
   <223> "Aib=2-amino-isobutyric acid"
<220>
   <221> BINDING
   <222> (3)..(4)
   <223> "Aib=2-amino-isobutyric acid"
<220>
   <221> BINDING
   <222> (4)..(5)
   <223> "Aib=2-amino-isobutyric acid"
<220>
   <221> BINDING
   <222> (5)..(6)
   <223> "Aib=2-amino-isobutyric acid"
<220>
   <221> BINDING
   <222> (6)..(7)
   <223> "Aib=2-amino-isobutyric acid"
<220>
   <221> BINDING
   <222> (7)..(8)
   <223> "Aib=2-amino-isobutyric acid"
<220>
   <221> BINDING
   <222> (8)..(9)
   <223> "Aib=2-amino-isobutyric acid"
<220>
   <221> BINDING
   <222> (9)..(10)
   <223> "Aib=2-amino-isobutyric acid"
<220>
   <221> MOD_RES
   <222> (10)..(10)
   <223> AMIDATION
<400> 13
   Lys Gly Lys Gly Lys Gly Lys Gly Lys Gly
   1               5                   10
<210> 14
   <211> 10
   <212> PRT
   <213> artificial sequence
<220>
   <223> artificial
<220>
   <221> MOD RES
   <222> (1)..(1)
   <223> ACETYLATION
<220>
   <221> BINDING
   <222> (1)..(2)
   <223> "Aib=2-amino-isobutyric acid"
<220>
   <221> BINDING
   <222> (3)..(4)
   <223> "Aib=2-amino-isobutyric acid"
<220>
   <221> BINDING
   <222> (5)..(6)
   <223> "Aib=2-amino-isobutyric acid"
<220>
   <221> BINDING
   <222> (7)..(8)
   <223> "Aib=2-amino-isobutyric acid"
<220>
   <221> BINDING
   <222> (9)..(10)
   <223> "Aib=2-amino-isobutyric acid"
<220>
   <221> MOD_RES
   <222> (10)..(10)
   <223> AMIDATION
<400> 14
   Lys Gly Lys Gly Lys Gly Lys Gly Lys Gly
   1               5                   10
<210> 15
   <211> 12
   <212> PRT
   <213> artificial sequence
<220>
   <223> artificial
<220>
   <221> BINDING
   <222> (1)..(1)
   <223> "Ac-Aib= acetyl-2-amino-isobutyric acid"
<220>
   <221> BINDING
   <222> (1)..(2)
   <223> "Aib=2-amino-isobutyric acid"
<220>
   <221> BINDING
   <222> (2)..(3)
<220>
   <221> BINDING
   <222> (3)..(4)
   <223> "Aib=2-amino-isobutyric acid"
<220>
   <221> BINDING
   <222> (4)..(5)
   <223> "Aib=2-amino-isobutyric acid"
<220>
   <221> BINDING
   <222> (5)..(6)
   <223> "Aib=2-amino-isobutyric acid"
<220>
   <221> BINDING
   <222> (6)..(7)
   <223> "Aib=2-amino-isobutyric acid"
<220>
   <221> BINDING
   <222> (7)..(8)
   <223> "Aib=2-amino-isobutyric acid"
<220>
   <221> BINDING
   <222> (8)..(9)
   <223> "Aib=2-amino-isobutyric acid"
<220>
   <221> BINDING
   <222> (9)..(10)
   <223> "Aib=2-amino-isobutyric acid"
<220>
   <221> BINDING
   <222> (10)..(11)
   <223> "Aib=2-amino-isobutyric acid"
<220>
   <221> BINDING
   <222> (11)..(12)
   <223> "Aib=2-amino-isobutyric acid"
<220>
   <221> MOD_RES
   <222> (12)..(12)
   <223> AMIDATION
<400> 15
   Lys Gly Lys Gly Lys Gly Lys Gly Lys Gly Lys Gly
   1               5                   10
<210> 16
   <211> 12
   <212> PRT
   <213> artificial sequence
<220>
   <223> artificial
<220>
   <221> MOD_RES
   <222> (1)..(1)
   <223> ACETYLATION
<220>
   <221> BINDING
   <222> (1)..(2)
   <223> "Aib=2-amino-isobutyric acid"
<220>
   <221> BINDING
   <222> (3)..(4)
   <223> "Aib=2-amino-isobutyric acid"
<220>
   <221> BINDING
   <222> (5)..(6)
   <223> "Aib=2-amino-isobutyric acid"
<220>
   <221> BINDING
   <222> (7)..(8)
   <223> "Aib=2-amino-isobutyric acid"
<220>
   <221> BINDING
   <222> (9)..(10)
   <223> "Aib=2-amino-isobutyric acid"
<220>
   <221> BINDING
   <222> (11)..(12)
   <223> "Aib=2-amino-isobutyric acid"
<220>
   <221> MOD_RES
   <222> (12)..(12)
   <223> AMIDATION
<400> 16
   Lys Gly Lys Gly Lys Gly Lys Gly Lys Gly Lys Gly
   1               5                   10
<210> 17
   <211> 63
   <212> PRT
   <213> Homo sapiens
<400> 17
   Lys Gly Glu Gly Gly Phe Gly Gly Arg Gly Gly Gly Arg Gly Gly Phe
   1               5                   10                  15
   Gly Gly Arg Gly Gly Gly Arg Gly Gly Arg Gly Gly Phe Gly Gly Arg
               20                  25                  30
   Gly Arg Gly Gly Phe Gly Gly Arg Gly Gly Phe Arg Gly Gly Arg Gly
           35                  40                  45
   Gly Gly Gly Asp His Lys Pro Gln Gly Lys Lys Thr Lys Phe Glu
       50                  55                  60
<210> 18
   <211> 35
   <212> DNA
   <213> artificial seqence
<220>
   <223> artificial sequence= primer
<400> 18
   ccggaattca tggctaaaga aaaatttgag cgttc 35
<210> 19
   <211> 42
   <212> DNA
   <213> artificial sequence
<220>
   <223> artificial sequence=primer
<400> 19
   cgagtcgact tactcgataa ttttagctac aacaccagca cc 42
<210> 20
   <211> 34
   <212> PRT
   <213> artificial sequence
<220>
   <223> artificial
<400> 20
   Ala Lys Val Lys Asp Glu Pro Gln Arg Arg Ser Ala Arg Leu Ser Ala
   1               5                   10                  15
   Lys Pro Ala Pro Pro Lys Pro Glu Pro Lys Pro Lys Lys Ala Pro Ala
               20                  25                  30
   Lys Lys